# EUROPÄISCHE PATENTSCHRIFT

(11) **EP 1 444 351 B1**
(45) Veröffentlichungstag und Bekanntmachung des Hinweises auf die Patenterteilung: **06.08.2008**
(21) Anmeldenummer: 02783042.1
(22) Anmeldetag: 31.10.2002
(51) Int. Cl.: C12N 15/52

(54) **GENE, DIE FUER KOHLENSTOFFMETABOLISMUS- UND ENERGIEPRODUKTIONS-PROTEINE CODIEREN**
GENES ENCODING FOR CARBON METABOLISM AND ENERGY-PRODUCING PROTEINS
GENES CODANT POUR DES PROTEINES DU METABOLISME DU CARBONE ET DE PRODUCTION D'ENERGIE

(30) Priorität: 05.11.2001 DE 10154270
(43) Veröffentlichungstag der Anmeldung: 11.08.2004
(60) Teilanmeldung: 08100575.3
(73) Patentinhaber: Paik Kwang Industrial Co., Ltd., Jeollabuk-do (KR)
(72) Erfinder: ZELDER, Oskar, 67346 Speyer (DE); POMPEJUS, Markus, 67251 Freinsheim (DE); SCHRÖDER, Hartwig, 69226 Nussloch (DE); KRÖGER, Burkhard, 67117 Limburgerhof (DE); KLOPPROGGE, Corinna, 68239 Mannheim (DE); HABERHAUER, Gregor, 67117 Limburgerhof (DE)
(74) Vertreter: Maiwald Patentanwalts GmbH
(86) Internationale Anmeldenummer: PCT/EP2002/012135
(87) Internationale Veröffentlichungsnummer: WO 2003/040291

(56) Entgegenhaltungen:
- WO-A-01/00805
- WO-A-01/00844
- DATABASE EMBL, HEIDELBERG, BRD [Online] SEQ ID NO: 1689 aus EP1108790, 26. September 2001 (2001-09-26) " " Database accession no. AAH66654 XP002240540 -& EP 1 108 790 A (KYOWA HAKKO KOGYO CO., LTD.) 20. Juni 2001 (2001-06-20)
- DATABASE EMBL, HEIDELBERG, BRD [Online] SEQ ID NO: 5189 aus EP1108790, 26. September 2001 (2001-09-26) " " Database accession no. AAG91435 XP002240541 & EP 1 108 790 A (KYOWA HAKKO KOGYO CO., LTD.) 20. Juni 2001 (2001-06-20)

### SEQUENCE LISTING

<110> BASF Aktiengesellschaft
<120> Gene die für Kohlenstoffmetabolismus- und Energieproduktion-Proteine codieren
<130> O.Z. 0050/52973
<160> 116
<210> 1
   <211> 1978
   <212> DNA
   <213> Corynebacterium glutamicum
<220>
   <221> CDS
   <222> (101)..(1948)
   <223> RXA00149
<400> 1
   ttttcgtagg taaacacagg tgaaggcttt acaagcttgt gaactcccta cacaaaagca 60
   atccaatagc tatccataag caagagaaag taagtctacg ttg act gat ctc aca   115
                                            Leu Thr Asp Leu Thr
                                              1               5
   aag act gcg gtg ccc gag gaa ctt tca gag aac ctc gaa act tgg tac   163
   Lys Thr Ala Val Pro Glu Glu Leu Ser Glu Asn Leu Glu Thr Trp Tyr
                    10                  15                  20
   aag gct gtg gcc ggt gtt ttc gcg cgc aca cag aaa aaa gac atc ggc   211
   Lys Ala Val Ala Gly Val Phe Ala Arg Thr Gln Lys Lys Asp Ile Gly
                25                  30                  35
   gac att gcc gta gat gtg tgg aag aaa ctc atc gtc act aca ccg gat   259
   Asp Ile Ala Val Asp Val Trp Lys Lys Leu Ile Val Thr Thr Pro Asp
            40                  45                  50
   ggt gtt gat atc aat ccg ctg tac acc aga gca gat gag tcc cag agg   307
   Gly Val Asp Ile Asn Pro Leu Tyr Thr Arg Ala Asp Glu Ser Gln Arg
        55                  60                  65
   aaa ttc act gag gtt cct ggt gag ttt ccc ttc act agg gga acc act   355
   Lys Phe Thr Glu Val Pro Gly Glu Phe Pro Phe Thr Arg Gly Thr Thr
    70                  75                  80                  85
   gtt gat ggt gaa cgc gtt ggt tgg ggt gtt act gag act ttc gga cat   403
   Val Asp Gly Glu Arg Val Gly Trp Gly Val Thr Glu Thr Phe Gly His
                    90                  95                 100
   gac agc ccg aag aat atc aac gct gcg gtg ctg aat gct ctg aat tct   451
   Asp Ser Pro Lys Asn Ile Asn Ala Ala Val Leu Asn Ala Leu Asn Ser
               105                 110                 115
   ggc acc acc aca ttg ggt ttt gag ttc tct gag gaa ttc acg gca gct   499
   Gly Thr Thr Thr Leu Gly Phe Glu Phe Ser Glu Glu Phe Thr Ala Ala
           120                 125                 130
   gat ctt aaa gtt gct ctc gaa ggc gtg tat ctc aac atg gct ccg ttg   547
   Asp Leu Lys Val Ala Leu Glu Gly Val Tyr Leu Asn Met Ala Pro Leu
       135                 140                 145
   ctg att cat gcg ggt gga tcc acg tca gag gtt gca gcg gcg ttg tat      595
   Leu Ile His Ala Gly Gly Ser Thr Ser Glu Val Ala Ala Ala Leu Tyr
   150                 155                 160                 165
   acg ttg gcg gag gaa gcc gga acg ttt ttt gct gcg ttg acc ttg ggt      643
   Thr Leu Ala Glu Glu Ala Gly Thr Phe Phe Ala Ala Leu Thr Leu Gly
                   170                 175                 180
   tct cgt cct ttg acg gcg cag gtt gat ggt tcg cac agt gac acc att      691
   Ser Arg Pro Leu Thr Ala Gln Val Asp Gly Ser His Ser Asp Thr Ile
               185                 190                 195
   gaa gaa gca gtt cag ttg gca gtg aat gct tcc aag cgt gcg aat gtg      739
   Glu Glu Ala Val Gln Leu Ala Val Asn Ala Ser Lys Arg Ala Asn Val
           200                 205                 210
   cgc gct atc ttg gtg gat ggt tcc agt ttt tcc aac cag ggc gcg tcg      787
   Arg Ala Ile Leu Val Asp Gly Ser Ser Phe Ser Asn Gln Gly Ala Ser
       215                 220                 225
   gat gct caa gaa att ggt cta agt atc gcc gcc ggt gtg gat tat gtc      835
   Asp Ala Gln Glu Ile Gly Leu Ser Ile Ala Ala Gly Val Asp Tyr Val
   230                 235                 240                 245
   cgt cgc ttg gtc gat gca ggc ctt tcc acg gaa gct gca ctt aag cag      883
   Arg Arg Leu Val Asp Ala Gly Leu Ser Thr Glu Ala Ala Leu Lys Gln
                   250                 255                 260
   gtg gcg ttc cgt ttt gcg gtc acc gat gag cag ttc gcg cag att tct      931
   Val Ala Phe Arg Phe Ala Val Thr Asp Glu Gln Phe Ala Gln Ile Ser
               265                 270                 275
   aag ctg cgt gtg gct cga cgt ctg tgg gcc agg gtg tgt gag gtg ctt      979
   Lys Leu Arg Val Ala Arg Arg Leu Trp Ala Arg Val Cys Glu Val Leu
           280                 285                 290
   ggt ttt cca gag ctg gcc gta gca cca cag cat gcg gtg act gca cga      1027
   Gly Phe Pro Glu Leu Ala Val Ala Pro Gln His Ala Val Thr Ala Arg
       295                 300                 305
   gcg atg ttt agc cag cgt gat ccg tgg gtg aat atg ctg cgc agt act      1075
   Ala Met Phe Ser Gln Arg Asp Pro Trp Val Asn Met Leu Arg Ser Thr
   310                 315                 320                 325
   gtt gca gct ttc gct gca ggc gtc ggt gga gca acc gat gtg gag gtt      1123
   Val Ala Ala Phe Ala Ala Gly Val Gly Gly Ala Thr Asp Val Glu Val
                   330                 335                 340
   cgt act ttt gat gat gcg atc cca gat gga gtt cct gga gtg tcg agg      1171
   Arg Thr Phe Asp Asp Ala Ile Pro Asp Gly Val Pro Gly Val Ser Arg
               345                 350                 355
   aat ttc gct cac cgc atc gcg cgc aat act aat ttg ttg ttg cta gaa      1219
   Asn Phe Ala His Arg Ile Ala Arg Asn Thr Asn Leu Leu Leu Leu Glu
           360                 365                 370
   gag tca cat ctt ggt cac gtg gtt gat cct gct ggt gga tca tat ttc      1267
   Glu Ser His Leu Gly His Val Val Asp Pro Ala Gly Gly Ser Tyr Phe
       375                 380                 385
   gtg gag agc ttc acc gat gat cta gcg gag aag gcg tgg gct gtg ttc      1315
   Val Glu Ser Phe Thr Asp Asp Leu Ala Glu Lys Ala Trp Ala Val Phe
   390                 395                 400                 405
   agt ggc atc gaa gct gag ggc gga tac agt gca gct tgt gca tcc ggc    1363
   Ser Gly Ile Glu Ala Glu Gly Gly Tyr Ser Ala Ala Cys Ala Ser Gly
                   410                 415                 420
   acg gtg act gcc atg ctt gat cag acg tgg gag cag act cgc gct gat    1411
   Thr Val Thr Ala Met Leu Asp Gln Thr Trp Glu Gln Thr Arg Ala Asp
               425                 430                 435
   gtg gcg tcg aga aag aag aag ctc act gga att aat gag ttc ccg aac    1459
   Val Ala Ser Arg Lys Lys Lys Leu Thr Gly Ile Asn Glu Phe Pro Asn
           440                 445                 450
   ttg gcg gag tct ccg ctg cca gct gat cgt cgg gta gaa cct gca ggt    1507
   Leu Ala Glu Ser Pro Leu Pro Ala Asp Arg Arg Val Glu Pro Ala Gly
       455                 460                 465
   gtg cgt cga tgg gca gcg gat ttt gaa gcg ctg cgc aat cgt tcg gat    1555
   Val Arg Arg Trp Ala Ala Asp Phe Glu Ala Leu Arg Asn Arg Ser Asp
   470                 475                 480                 485
   gct ttc ttg gaa aag aac ggc gcg agg cca cag atc acg atg att cct    1603
   Ala Phe Leu Glu Lys Asn Gly Ala Arg Pro Gln Ile Thr Met Ile Pro
                   490                 495                 500
   ctg gga ccg ttg tcc aag cac aat att cgc act ggt ttt act tcc aac    1651
   Leu Gly Pro Leu Ser Lys His Asn Ile Arg Thr Gly Phe Thr Ser Asn
               505                 510                 515
   ctg ttg gct tcc ggt ggc att gaa gca atc aac ccg ggt caa ctt gtt    1699
   Leu Leu Ala Ser Gly Gly Ile Glu Ala Ile Asn Pro Gly Gln Leu Val
           520                 525                 530
   ccc ggc act gac gct ttt gca gaa gct gca cag gcc gca ggc att gta    1747
   Pro Gly Thr Asp Ala Phe Ala Glu Ala Ala Gln Ala Ala Gly Ile Val
       535                 540                 545
   gtg gtg tgt gga acg gac caa gag tat gcc gaa acg ggg gag gga gcc    1795
   Val Val Cys Gly Thr Asp Gln Glu Tyr Ala Glu Thr Gly Glu Gly Ala
   550                 555                 560                 565
   gtc gaa aag ctc cgc gaa gcg ggc gtt gag cgc atc ctg ctt gct ggc    1843
   Val Glu Lys Leu Arg Glu Ala Gly Val Glu Arg Ile Leu Leu Ala Gly
                   570                 575                 580
   gcg ccg aag agc ttt gag ggc agc gcg cat gcg ccc gat ggt tat ttg    1891
   Ala Pro Lys Ser Phe Glu Gly Ser Ala His Ala Pro Asp Gly Tyr Leu
               585                 590                 595
   aac atg aca att gat gcc gcg gcg acg ctg gct gac ctg cta gat gct    1939
   Asn Met Thr Ile Asp Ala Ala Ala Thr Leu Ala Asp Leu Leu Asp Ala
           600                 605                 610
   ttg gga gct taaatcatga cgtcgatccc taatttttca                       1978
   Leu Gly Ala
       615
<210> 2
   <211> 616
   <212> PRT
   <213> Corynebacterium glutamicum
<400> 2
   Leu Thr Asp Leu Thr Lys Thr Ala Val Pro Glu Glu Leu Ser Glu Asn
   1               5                   10                  15
   Leu Glu Thr Trp Tyr Lys Ala Val Ala Gly Val Phe Ala Arg Thr Gln
               20                  25                  30
   Lys Lys Asp Ile Gly Asp Ile Ala Val Asp Val Trp Lys Lys Leu Ile
           35                  40                  45
   Val Thr Thr Pro Asp Gly Val Asp Ile Asn Pro Leu Tyr Thr Arg Ala
       50                  55                  60
   Asp Glu Ser Gln Arg Lys Phe Thr Glu Val Pro Gly Glu Phe Pro Phe
   65                  70                  75                  80
   Thr Arg Gly Thr Thr Val Asp Gly Glu Arg Val Gly Trp Gly Val Thr
                   85                  90                  95
   Glu Thr Phe Gly His Asp Ser Pro Lys Asn Ile Asn Ala Ala Val Leu
               100                 105                 110
   Asn Ala Leu Asn Ser Gly Thr Thr Thr Leu Gly Phe Glu Phe Ser Glu
           115                 120                 125
   Glu Phe Thr Ala Ala Asp Leu Lys Val Ala Leu Glu Gly Val Tyr Leu
       130                 135                 140
   Asn Met Ala Pro Leu Leu Ile His Ala Gly Gly Ser Thr Ser Glu Val
   145                 150                 155                 160
   Ala Ala Ala Leu Tyr Thr Leu Ala Glu Glu Ala Gly Thr Phe Phe Ala
                   165                 170                 175
   Ala Leu Thr Leu Gly Ser Arg Pro Leu Thr Ala Gln Val Asp Gly Ser
               180                 185                 190
   His Ser Asp Thr Ile Glu Glu Ala Val Gln Leu Ala Val Asn Ala Ser
           195                 200                 205
   Lys Arg Ala Asn Val Arg Ala Ile Leu Val Asp Gly Ser Ser Phe Ser
       210                 215                 220
   Asn Gln Gly Ala Ser Asp Ala Gln Glu Ile Gly Leu Ser Ile Ala Ala
   225                 230                 235                 240
   Gly Val Asp Tyr Val Arg Arg Leu Val Asp Ala Gly Leu Ser Thr Glu
                   245                 250                 255
   Ala Ala Leu Lys Gln Val Ala Phe Arg Phe Ala Val Thr Asp Glu Gln
               260                 265                 270
   Phe Ala Gln Ile Ser Lys Leu Arg Val Ala Arg Arg Leu Trp Ala Arg
           275                 280                 285
   Val Cys Glu Val Leu Gly Phe Pro Glu Leu Ala Val Ala Pro Gln His
       290                 295                 300
   Ala Val Thr Ala Arg Ala Met Phe Ser Gln Arg Asp Pro Trp Val Asn
   305                 310                 315                 320
   Met Leu Arg Ser Thr Val Ala Ala Phe Ala Ala Gly Val Gly Gly Ala
                   325                 330                 335
   Thr Asp Val Glu Val Arg Thr Phe Asp Asp Ala Ile Pro Asp Gly Val
                       340                 345                 350
   Pro Gly Val Ser Arg Asn Phe Ala His Arg Ile Ala Arg Asn Thr Asn
               355                 360                 365
   Leu Leu Leu Leu Glu Glu Ser His Leu Gly His Val Val Asp Pro Ala
           370                 375                 380
   Gly Gly Ser Tyr Phe Val Glu Ser Phe Thr Asp Asp Leu Ala Glu Lys
   385                 390                 395                 400
   Ala Trp Ala Val Phe Ser Gly Ile Glu Ala Glu Gly Gly Tyr Ser Ala
                   405                 410                 415
   Ala Cys Ala Ser Gly Thr Val Thr Ala Met Leu Asp Gln Thr Trp Glu
               420                 425                 430
   Gln Thr Arg Ala Asp Val Ala Ser Arg Lys Lys Lys Leu Thr Gly Ile
           435                 440                 445
   Asn Glu Phe Pro Asn Leu Ala Glu Ser Pro Leu Pro Ala Asp Arg Arg
       450                 455                 460
   Val Glu Pro Ala Gly Val Arg Arg Trp Ala Ala Asp Phe Glu Ala Leu
   465                 470                 475                 480
   Arg Asn Arg Ser Asp Ala Phe Leu Glu Lys Asn Gly Ala Arg Pro Gln
                   485                 490                 495
   Ile Thr Met Ile Pro Leu Gly Pro Leu Ser Lys His Asn Ile Arg Thr
               500                 505                 510
   Gly Phe Thr Ser Asn Leu Leu Ala Ser Gly Gly Ile Glu Ala Ile Asn
           515                 520                 525
   Pro Gly Gln Leu Val Pro Gly Thr Asp Ala Phe Ala Glu Ala Ala Gln
       530                 535                 540
   Ala Ala Gly Ile Val Val Val Cys Gly Thr Asp Gln Glu Tyr Ala Glu
   545                 550                 555                 560
   Thr Gly Glu Gly Ala Val Glu Lys Leu Arg Glu Ala Gly Val Glu Arg
                   565                 570                 575
   Ile Leu Leu Ala Gly Ala Pro Lys Ser Phe Glu Gly Ser Ala His Ala
               580                 585                 590
   Pro Asp Gly Tyr Leu Asn Met Thr Ile Asp Ala Ala Ala Thr Leu Ala
           595                 600                 605
   Asp Leu Leu Asp Ala Leu Gly Ala
       610                 615

## Beschreibung

### Hintergrund der Erfindung

Bestimmte Produkte und Nebenprodukte von natürlich-vorkommenden Stoffwechselprozessen in Zellen eignen sich für viele Industriezweige, einschließlich der Nahrungsmittel-, Futtermittel-, Kosmetik- und pharmazeutischen Industrie. Diese Moleküle, die man gemeinsam als "Feinchemikalien" bezeichnet, umfassen organische Säuren, sowohl proteinogene als auch nicht-proteinogene Aminosäuren, Nukleotide und Nukleoside, Lipide und Fettsäuren, Diole, Kohlehydrate, aromatische Verbindungen, Vitamine und Cofaktoren sowie Enzyme. Ihre Produktion erfolgt am besten mittels Anzucht von Bakterien im Großmaßstab, die entwickelt wurden, um große Mengen des jeweils gewünschten Moleküls zu produzieren und sezernieren. Ein für diesen Zweck besonders geeigneter Organismus ist *Corynebacterium glutamicum,* ein gram-positives, nicht-pathogenes Bakterium. Über Stammselektion ist eine Reihe von Mutantenstämmen entwickelt worden, die ein Sortiment wünschenswerter Verbindungen produzieren. Die Auswahl von Stämmen, die hinsichtlich der Produktion eines bestimmten Moleküls verbessert sind, ist jedoch ein zeitaufwendiges und schwieriges Verfahren. WO 01/00844 beschreibt eine Methylmalonyl-COA Mutase aus Corynebakterium.

### Zusammenfassung der Erfindung

Diese Erfindung stellt neuartige Nukleinsäuremoleküle bereit, die sich zur Identifizierung oder Klassifizierung von *Corynebacterium glutamicum* oder verwandten Bakterienarten verwenden lassen. *C. glutamicum* ist ein gram-positives, aerobes Bakterium, das in der Industrie für die Produktion im Großmaßstab einer Reihe von Feinchemikalien und auch zum Abbau von Kohlenwasserstoffen (wie bspw. bei verschüttetem Rohöl) und zur Oxidation von Terpenoiden gemeinhin verwendet wird. Die Nukleinsäuremoleküle können daher zum Identifizieren von Mikroorganismen eingesetzt werden, die sich zur Produktion von Feinchemikalien, bspw. durch Fermentationsverfahren, verwenden lassen. *C. glutamicum* selbst ist zwar nicht-pathogen, jedoch ist es mit anderen Corynebacterium-Arten, wie Co*rynebacterium diphtheriae* (dem Erreger der Diphtherie) verwandt, die bedeutende Pathogene beim Menschen sind. Die Fähigkeit, das Vorhandensein von Corynebacterium-Arten zu identifizieren, kann daher auch eine signifikante klinische Bedeutung haben, z.B. bei diagnostischen Anwendungen. Diese Nukleinsäuremoleküle können zudem als Bezugspunkte zur Kartierung des *C. glutamicum*-Genoms oder von Genomen verwandter Organismen dienen.

Diese neuen Nukleinsäuremoleküle codieren Proteine, die hier als Proteine des Zuckermetabolismus und der oxidativen Phosphorylierung (sugar metabolism and oxidative phosphorylation (SMP) proteins) bezeichnet werden. Diese SMP-Proteine haben bspw. eine Funktion beim Stoffwechsel von Kohlenstoffverbindungen, wie Zukkern, oder bei der Erzeugung von Energiemolekülen durch Prozesse, wie die oxidative Phosphorylierung, in *C*. *glutamicum.* Aufgrund der Verfügbarkeit von in *Corynebacterium glutamicum* verwendbaren Klonierungsvektoren, wie bspw. offenbart in Sinskey et al., US-Patent Nr. 4 649 119, und von Techniken zur genetischen Manipulation von *C*. *glutamicum* und den verwandten *Brevibacterium*-Arten (z.B. *lactofermentum*) (Yoshihama et al., J. Bacteriol. 162:591-597 (1985); Katsumata et al., J. Bacteriol. 159:306-311 (1984); und Santamaria et al. J. Gen. Microbiol. 130:2237-2246 (1984)), lassen sich die erfindungsgemäßen Nukleinsäuremoleküle zur genetischen Manipulation dieses Organismus verwenden, um ihn als Produzenten von einer oder mehreren Feinchemikalien besser und effizienter zu machen. Diese verbesserte Produktion oder Effizienz der Produktion einer Feinchemikalie kann aufgrund einer direkten Auswirkung der Manipulation eines erfindungsgemäßen Gens oder aufgrund einer indirekten Auswirkung einer solchen Manipulation erfolgen.

Es gibt eine Reihe von Mechanismen, durch die die Veränderung eines erfindungsgemäßen SMP-Proteins die Ausbeute, Produktion und/oder Effizienz der Produktion einer Feinchemikalie aus einem *C*. *glutamicum*-Stamm, der dieses veränderte Protein enthält, direkt beeinflussen kann. Der Abbau energiereicher Kohlenstoffmoleküle, bspw. von Zuckern, und die Umwandlung von Verbindungen, wie NADH und FADH₂ in Verbindungen mit energiereichen Phosphatbindungen über die oxidative Phosphorylierung führt zu einer Reihe von Verbindungen, die selbst wünschenwerte Feinchemikalien sein können, wie Pyruvat, ATP, NADH, und zu einer Reihe von Zuckerzwischenverbindungen. Ferner werden die Energiemoleküle (wie ATP) und die Reduktionsäquivalente (wie NADH oder NADPH), die durch diese Stoffwechselwege produziert werden, in der Zelle zum Antreiben von Reaktionen verwendet, die ansonsten energetisch ungünstig wären. Zu solchen ungünstigen Reaktionen gehören viele Biosynthesewege von Feinchemikalien. Durch die Verbesserung der Fähigkeit der Zelle, einen bestimmten Zucker zu nutzen (z.B. durch Manipulation der Gene, die am Abbau und an der Umwandlung des Zuckers in Energie für die Zelle beteiligt sind) läßt sich die Menge an Energie, die verfügbar ist, damit ungünstige und trotzdem gewünschte Stoffwechselreaktion (z.B. die Biosynthese einer gewünschten Feinchemikalie) auftreten, vergrößern.

Die Mutagenese von einem oder mehreren erfindungsgemäßen SMP-Proteinen kann auch zu SMP-Proteinen mit geänderten Aktivitäten führen, die indirekt die Produktion einer oder mehrere gewünschten Feinchemikalien aus *C*. *glutamicum* beeinflussen. Beispielsweise kann man durch Erhöhen der Effizienz der Nutzung eines oder mehrerer Zucker (so daß die Umwandlung des Zuckers in nutzbare Energiemoleküle verbessert ist) oder durch Erhöhen der Effizienz der Umwandlung von Reduktionsäquivalenten in nutzbare Energiemoleküle (z.B. durch Verbesserung der Effizienz der oxidativen Phosphorylierung oder der Aktivität der ATP-Synthase) die Menge dieser energiereichen Verbindungen, die für die Zellen zum Antrieb normalerweise ungünstiger Stoffwechselprozesse verfügbar ist, steigern. Zu diesen Prozessen gehört der Aufbau der Zellwände, die Transkription, Translation, die Biosynthese von Verbindungen, die für das Wachstum und die Teilung von Zellen nötig sind (z.B. Nukleotide, Aminosäuren, Vitamine, Lipide usw.) (Lengeler et al. (1999) Biology of Prokaryotes, Thieme Verlag: Stuttgart, S. 88-109; 913-918; 875-899). Durch Verbessern von Wachstum und Vermehrung dieser veränderten Zellen ist es möglich, die Lebensfähigkeit der Zellen in Kulturen im Großmaßstab zu steigern und auch ihre Teilungsrate zu verbessern, daß eine vergleichsweise größere Anzahl Zellen in der Fermenterkultur überleben kann. Die Ausbeute, Produktion oder Effizienz der Produktion kann zumindest aufgrund der Anwesenheit einer größeren Anzahl lebensfähiger Zellen, die jeweils die gewünschte Feinchemikalie produzieren, erhöht werden. Auch viele der Abbauprodukte, die während des Zukkerstoffwechsels produziert werden, werden von der Zelle als Vorstufen oder Zwischenprodukte bei der Produktion anderer wünschenswerter Produkte, bspw. von Feinchemikalien, verwendet. So sollte durch Steigerung der Fähigkeit der Zelle, Zucker zu metabolisieren, die Anzahl dieser Abbauprodukte, die für die Zelle für andere Prozesse verfügbar sind, gesteigert werden.

Diese Erfindung stellt neue Nukleinsäuremoleküle bereit, gemäß Anspruch 1 die die hier als SMP-Proteine bezeichneten Proteine codieren, welche bspw. eine Funktion ausüben können, die am Stoffwechsel von Kohlenstoffverbindungen, wie Zuckern, und an der Erzeugung von Energiemolekülen durch Prozesse, wie oxidative Phosphprylierung, in *Corynebacterium glutamicum* beteiligt sind. Nukleinsäuremoleküle, die ein SMP-Protein codieren, werden hier als SMP-Nukleinsäuremoleküle bezeichnet. Bei einer bevorzugten Ausführungsform ist das SMP-Protein an der Umwandlung von Kohlenstoffmolekülen und deren Abbauprodukten in Energie beteiligt, die von der Zelle für Stoffwechselvorgänge verwendet wird. Beispiele für solche Proteine sind diejenigen, die von den in Tabelle 1 angegebenen Genen codiert werden.

Ein Aspekt der Erfindung betrifft folglich isolierte Nukleinsäuremoleküle (bspw. cDNAs), umfassend eine Nukleotidsequenz, die ein SMP-Protein codiert. Bei besonders bevorzugten Ausführungsformen umfaßt das isolierte Nukleinsäuremolekül eine der in Anhang A aufgeführten Nukleotidsequenzen oder den codierenden Bereich einer dieser Nukleotidsequenzen oder ein Komplement davon. In anderen bevorzugten Ausführungsformen codiert das isolierte Nukleinsäuremolekül eine der in Anhang B aufgeführten Aminosäuresequenzen. Die bevorzugten erfindungsgemäßen SMP-Proteine besitzen ebenfalls vorzugsweise mindestens eine der hier beschriebenen SMP-Aktivitäten.

Als Anhang A werden im folgenden die Nukleinsäuresequenzen des Sequenzprotokolls zusammen mit den in Tabelle 1 beschriebenen Sequenzveränderungen an der jeweiligen Position definiert.

Als Anhang B werden im folgenden die Polypeptidsequenzen des Sequenzprotokolls zusammen mit den in Tabelle 1 beschriebenen Sequenzveränderungen an der jeweiligen Position definiert.

Ein weiterer Aspekt der Erfindung betrifft Vektoren, bspw. rekombinante Expressionsvektoren, die die erfindungsgemäßen Nukleinsäuremoleküle enthalten, und Wirtszellen, in die diese Vektoren eingebracht worden sind. Bei einer Ausführungsform wird diese Wirtszelle zur Herstellung eines SMP-Proteins verwendet, indem die Wirtszelle in einem geeigneten Medium gezüchtet wird. Das SMP-Protein kann dann aus dem Medium oder der Wirtszelle isoliert werden.

Ein weiterer Aspekt der Erfindung betrifft einen genetisch veränderten Mikroorganismus, bei dem ein SMP-Gen eingebracht oder verändert worden ist. Das Genom des Mikroorganismus ist bei einer Ausführungsform durch Einbringen mindestens eines erfindungsgemäßen Nukleinsäuremoleküls verändert worden, das die mutierte SMP-Sequenz als Transgen codiert. Bei einer anderen Ausführungsform ist ein endogenes SMP-Gen im Genom des Mikroorganismus durch homologe Rekombination mit einem veränderten SMP-Gen verändert, z.B. funktionell disruptiert, worden. Der Mikroorganismus gehört bei einer bevorzugten Ausführungsform zur Gattung *Corynebacterium* oder *Brevibacterium,* wobei *Corynebacterium glutamicum* besonders bevorzugt ist. Der Mikroorganismus wird in einer bevorzugten Ausführungsform auch zur Herstellung einer gewünschten Verbindung, wie einer Aminosäure verwendet, wobei Lysin besonders bevorzugt ist.

Eine weitere bevorzugte Ausführungsform sind Wirtszellen, die mehr als eine der in Anhang A beschriebenen Nukleinsäuremoleküle besitzen. Solche Wirtszellen lassen sich auf verschiedene dem Fachmann bekannte Wege herstellen. Beispielsweise können sie durch Vektoren, die mehrere der erfindungsgemäßen Nukleinsäuremoleküle tragen, transfiziert werden. Es ist aber auch möglich mit einem Vektor jeweils ein erfindungsgemäßes Nukleinsäuremolekül in die Wirtszelle einzubringen und deshalb mehrere Vektoren entweder gleichzeitig oder zeitlich abgestuft einzusetzen. Es können somit Wirtszellen konstruiert werden, die zahlreiche, bis zu mehreren Hundert der erfindungsgemäßen Nukleinsäuresequenzen tragen. Durch eine solche Akkumulation lassen sich häufig überadditive Effekte auf die Wirtszelle hinsichtlich der Feinchemikalien-Produktivität erzielen.

Ein weiterer Aspekt der Erfindung betrifft ein isoliertes SMP-Protein oder einen biologisch aktiven Abschnitt, davon. Das isolierte SMP-Protein oder sein Abschnitt kann in einer bevorzugten Ausführungsform eine am Stoffwechsel von Kohlenstoffverbindungen, wie Zuckern, oder an der Erzeugung von Energiemolekülen (z.B. ATP) durch Prozesse, wie oxidative Phosphorylierung, beteiligte Funktion in *Corynebacterium glutami*cum beteiligte Funktion ausüben. Bei einer weiteren bevorzugten Ausführungsform ist das isolierte SMP-Protein oder ein Abschnitt davon hinreichend homolog zu einer Aminosäuresequenz von Anhang B, so daß das Protein oder sein Abschnitt die Fähigkeit behält, eine am Stoffwechsel von Kohlenstoffverbindungen, wie Zuckern, oder an der Erzeugung von Energiemolekülen (z.B. ATP) durch Prozesse, wie oxidative Phosphorylierung, beteiligte Funktion in Co*rynebacterium glutamicum* auszuüben.

Die Erfindung betrifft zudem ein isoliertes SMP-Proteinpräparat. Das SMP-Protein umfaßt bei bevorzugten Ausführungsformen eine Aminosäuresequenz aus Anhang B. Bei einer weiteren bevorzugten Ausführungsform betrifft die Erfindung ein isoliertes Vollängenprotein, das zu einer vollständigen Aminosäuresequenz aus Anhang B (welche von einem offenen Leseraster in Anhang A codiert wird) im wesentlichen homolog ist.

Das SMP-Polypeptid oder ein biologisch aktiver Abschnitt davon kann mit einem Nicht-SMP-Polypeptid funktionsfähig verbunden werden, damit ein Fusionsprotein entsteht. Dieses Fusionsprotein hat bei bevorzugten Ausführungsformen eine andere Aktivität als das SMP-Protein allein. Bei anderen bevorzugten Ausführungsformen übt dieses Fusionsprotein eine am Stoffwechsel von Kohlenstoffverbindungen, wie Zuckern, oder an der Erzeugung von Energiemolekülen (z.B. ATP) durch Prozesse, wie oxidative Phosphorylierung, beteiligte Funktion in *Corynebacterium glutamicum* aus. Die Integration dieses Fusionsproteins in eine Wirtszelle moduliert bei besonders bevorzugten Ausführungsformen die Produktion einer gewünschten Verbindung von der Zelle.

Ein weiterer Aspekt der Erfindung betrifft ein Verfahren zur Herstellung einer Aminosäure. Das Verfahren sieht die Anzucht einer Zelle vor, die einen Vektor enthält, der die Expression eines erfindungsgemäßen SMP-Nukleinsäuremoleküls bewirkt, so daß eine Feinchemikalie produziert wird. Dieses Verfahren umfaßt bei einer bevorzugten Ausführungsform zudem den Schritt der Gewinnung einer Zelle, die einen solchen Vektor enthält, wobei die Zelle mit einem Vektor transfiziert ist, der die Expression einer SMP-Nukleinsäure bewirkt. Dieses Verfahren umfaßt bei einer weiteren bevorzugten Ausführungsform zudem den Schritt, bei dem die Feinchemikalie aus der Kultur gewonnen wird. Die Zelle gehört bei einer besonders bevorzugten Ausführungsform zur Gattung *Corynebacterium* oder *Brevibacterium.*

Beschrieben werden Verfahren zur Modulation der Produktion eines Moleküls von einem Mikroorganismus. Diese Verfahren umfassen das Zusammenbringen der Zelle mit einer Substanz, die die SMP-Proteinaktivität oder die SMP-Nukleinsäure-Expression moduliert, so daß eine zellassoziierte Aktivität verglichen mit der gleichen Aktivität bei Fehlen der Substanz verändert wird. Die Zelle wird bei einer bevorzugten Ausführungsform hinsichtlich eines oder mehrerer Kohlenstoff-Stoffwechselwege von *C. glutamicum* oder der Erzeugung von Energie durch Prozesse, wie oxidative Phosphorylierung moduliert, so daß die Ausbeute oder die Geschwindigkeit der Produktion einer gewünschten Feinchemikalie durch diesen Mikroorganismus verbessert wird. Die Substanz, die die SMP-Proteinaktivität moduliert, kann eine Substanz sein, die die SMP-Proteinaktivität oder die SMP-Nukleinsäure-Expression stimuliert. Beispiele für Substanzen, die die SMP-Proteinaktivität oder SMP-Nukleinsäureexpression stimulieren, umfassen kleine Moleküle, aktive SMP-Proteine und Nukleinsäuren, die SMP-Proteine codieren und in die Zelle eingebracht worden sind. Beispiele für Substanzen, die die SMP-Aktivität oder -Expression hemmen, umfassen kleine Moleküle und SMP-Antisense-Nukleinsäuremoleküle.

Des weiteren beschrieben werden Verfahren zur Modulation der Ausbeuten einer gewünschten Verbindung aus einer Zelle, umfassend das Einbringen eines SMP-Wildtyp- oder -Mutantengens in eine Zelle, das entweder auf einem gesonderten Plasmid bleibt oder in das Genom der Wirtszelle integriert wird. Die Integration in das Genom kann zufallsgemäß oder durch homologe Rekombination erfolgen, so daß das native Gen durch die integrierte Kopie ersetzt wird, was die Produktion der gewünschten Verbindung von der zu modulierenden Zelle hervorruft. Diese Ausbeuten sind bei einer bevorzugten Ausführungsform erhöht. Bei einer weiteren bevorzugten Ausführungsform ist die Chemikalie eine Feinchemikalie, die in einer besonders bevorzugten Ausführungsform eine Aminosäure ist. Diese Aminosäure ist in einer besonders bevorzugten Ausführungsform L-Lysin.

### Eingehende Beschreibung der Erfindung

Die vorliegende Erfindung stellt SMP-Nukleinsäure- und -Proteinmoleküle gemäß Anspruch 1 bereit, die am Stoffwechsel von Kohlenstoffverbindungen, wie Zuckern, und an der Erzeugung von Energiemolekülen durch Prozesse, wie oxidative Phosphorylierung, in *Corynebacterium glutamicum* beteiligt sind. Die erfindungsgemäßen Moleküle lassen sich bei der Modulation der Produktion von Feinchemikalien von Mikroorganismen, wie *C*. *glutamicum,* entweder direkt (z.B. wenn die Überexpression oder Optimierung eines Proteins des Glykolysewegs eine direkte Auswirkung auf Ausbeute, Produktion und/oder Effizienz der Produktion von z.B. Pyruvat aus modifizierten *C*. *gluta*micum hat) oder durch indirekte Auswirkung verwenden, was dennoch zu einem Anstieg der Ausbeute, Produktion und/oder Effizienz der Produktion der gewünschten Verbindung führt (z.B. wenn die Modulation von an der oxidativen Phosphorylierung beteiligten Proteinen zu Änderungen in der Menge der Energie führt, die zur Durchführung notwendiger Stoffwechselprozesse und anderer Zellfunktionen, wie Nukleinsäure- und Proteinbiosynthese und Transkription/Translation, verfügbar ist). Die erfindungsgemäßen Aspekte sind nachstehend weiter erläutert.

### I. Feinchemikalien

Der Begriff "Feinchemikalie" ist im Fachgebiet bekannt und beinhaltet Moleküle, die von einem Organismus produziert werden und in verschiedenen Industriezweigen Anwendungen finden, wie bspw., jedoch nicht beschränkt auf die pharmazeutische Industrie, die Landwirtschafts- und Kosmetikindustrie. Diese Verbindungen umfassen organische Säuren, wie Weinsäure, Itaconsäure und Diaminopimelinsäure, sowohl proteinogene als auch nicht-proteinogene Aminosäuren, Purin- und Pyrimidinbasen, Nukleoside und Nukleotide (wie bspw. beschrieben in Kuninaka, A. (1996) Nucleotides and related compounds, S. 561-612, in Biotechnology Bd. 6, Rehm et al., Hrsg. VCH: Weinheim und den darin enthaltenen Zitaten), Lipide, gesättigte und ungesättigte Fettsäuren (bspw. Arachidonsäure), Diole (bspw. Propandiol und Butandiol), Kohlehydrate (bspw. Hyaluronsäure und Trehalose), aromatische Verbindungen (bspw. aromatische Amine, Vanillin und Indigo), Vitamine und Cofaktoren (wie beschrieben in Ullmann's Encyclopedia of Industrial Chemistry, Bd. A27, "Vitamins", S. 443-613 (1996) VCH: Weinheim und den darin enthaltenen Zitaten; und Ong, A.S., Niki, E. und Packer, L. (1995) "Nutrition, Lipids, Health and Disease" Proceedings of the UNESCO/Confederation of Scientific and Technological Associations in Malaysia and the Society for Free Radical Research - Asien, abgehalten am 1.-3. Sept. 1994 in Penang, Malaysia, AOCS Press (1995)), Enzyme und sämtliche anderen von Gutcho (1983) in Chemicals by Fermentation, Noyes Data Corporation, ISBN: 0818805086 und den darin angegebenen Literaturstellen beschriebenen Chemikalien. Der Metabolismus und die Verwendungen bestimmter Feinchemikalien sind nachstehend weiter erläutert.

### A. Metabolismus und Verwendungen von Aminosäuren

Die Aminosäuren umfassen die grundlegenden Struktureinheiten sämtlicher Proteine und sind somit für die normalen Zellfunktionen in allen Organismen essentiell. Der Begriff "Aminosäure" ist im Fachgebiet bekannt. Die proteinogenen Aminosäuren, von denen es 20 Arten gibt, dienen als Struktureinheiten für Proteine, in denen sie über Peptidbindungen miteinander verknüpft sind, wohingegen die nicht-proteinogenen Aminosäuren (von denen Hunderte bekannt sind) gewöhnlich nicht in Proteinen vorkommen (siehe Ulmann's Encyclopedia of Industrial Chemistry, Bd. A2, S. 57-97 VCH: Weinheim (1985)). Die Aminosäuren können in der optischen D- oder L-Konfiguration vorliegen, obwohl L-Aminosäuren gewöhnlich der einzige Typ sind, den man in natürlich vorkommenden Proteinen vorfindet. Biosynthese- und Abbauwege von jeder der 20 proteinogenen Aminosäuren sind sowohl bei prokaryotischen als auch eukaryotischen Zellen gut charakterisiert (siehe bspw. Stryer, L., Biochemistry, 3. Auflage, S. 578-590 (1988)). Die "essentiellen" Aminosäuren (Histidin, Isoleucin, Leucin, Lysin, Methionin, Phenylalanin, Threonin, Tryptophan und Valin), so bezeichnet, weil sie aufgrund der Komplexität ihrer Biosynthese gewöhnlich mit der Ernährung aufgenommen werden müssen, werden durch einfache Biosynthesewege in die übrigen 11 "nichtessentiellen" Aminosäuren (Alanin, Arginin, Asparagin, Aspartat, Cystein, Glutamat, Glutamin, Glycin, Prolin, Serin und Tyrosin) umgewandelt. Höhere Tiere besitzen die Fähigkeit, einige dieser Aminosäuren zu synthetisieren, jedoch müssen die essentiellen Aminosäuren mit der Nahrung aufgenommen werden, damit eine normale Proteinsynthese stattfindet.

Abgesehen von ihrer Funktion bei der Proteinbiosynthese sind diese Aminosäuren interessante Chemikalien an sich, und man hat entdeckt, daß viele bei verschiedenen Anwendungen in der Nahrungsmittel-, Futter-, Chemie-, Kosmetik-, Landwirtschafts- und pharmazeutischen Industrie zum Einsatz kommen. Lysin ist nicht nur für die Ernährung des Menschen eine wichtige Aminosäure, sondern auch für monogastrische Tiere, wie Geflügel und Schweine. Glutamat wird am häufigsten als Geschmacksadditiv (Mononatriumglutamat, MSG) sowie weithin in der Nahrungsmittelindustrie verwendet, wie auch Aspartat, Phenylalanin, Glycin und Cystein. Glycin, L-Methionin und Tryptophan werden sämtlich in der pharmazeutischen Industrie verwendet. Glutamin, Valin, Leucin, Isoleucin, Histidin, Arginin, Prolin, Serin und Alanin werden in der pharmazeutischen Industrie und der Kosmetikindustrie verwendet. Threonin, Tryptophan und D-/L-Methionin sind weitverbreitete Futtermittelzusätze (Leuchtenberger, W. (1996) Amino acids - technical production and use, S. 466-502 in Rehm et al., (Hrsg.) Biotechnology Bd. 6, Kapitel 14a, VCH: Weinheim). Man hat entdeckt, daß sich diese Aminosäuren außerdem als Vorstufen für die Synthese von synthetischen Aminosäuren und Proteinen, wie N-Acetylcystein, S-Carboxymethyl-L-cystein, (S)-5-Hydroxytryptophan und anderen in Ulmann's Encyclopedia of Industrial Chemistry, Bd. A2, S. 57-97, VCH, Weinheim, 1985 beschriebenen Substanzen eignen.

Die Biosynthese dieser natürlichen Aminosäuren in Organismen, die sie produzieren können, bspw. Bakterien, ist gut charakterisiert worden (für einen Überblick der bakteriellen Aminosäure-Biosynthese und ihrer Regulation s. Umbarger, H.E. (1978) Ann. Rev. Biochem. 47:533-606). Glutamat wird durch reduktive Aminierung von α-Ketoglutarat, einem Zwischenprodukt im Citronensäure-Zyklus, synthetisiert. Glutamin, Prolin und Arginin werden jeweils nacheinander aus Glutamat erzeugt. Die Biosynthese von Serin erfolgt in einem Dreischritt-Verfahren, beginnt mit 3-Phosphoglycerat (einem Zwischenprodukt der Glykolyse) und ergibt nach Oxidations-, Transaminierungs- und Hydrolyseschritten diese Aminosäure. Cystein und Glycin werden jeweils aus Serin produziert, und zwar die erstere durch Kondensation von Homocystein mit Serin, und die letztere durch Übertragung des Seitenketten-β-Kohlenstoffätoms auf Tetrahydrofolat in einer durch Serin-Transhydroxymethylase katalysierten Reaktion. Phenylalanin und Tyrosin werden aus den Vorstufen des Glykolyse- und Pentosephosphatweges, Erythrose-4-phosphat und Phosphoenolpyruvat, in einem 9-Schritt-Biosyntheseweg synthetisiert, der sich nur in den letzten beiden Schritten nach der Synthese von Präphenat unterscheidet. Tryptophan wird ebenfalls aus diesen beiden Ausgangsmolekülen produziert, jedoch erfolgt dessen Synthese in einem 11-Schritt-Weg. Tyrosin läßt sich in einer durch Phenylalaninhydroxylase katalysierten Reaktion auch aus Phenylalanin herstellen. Alanin, Valin und Leucin sind jeweils Biosyntheseprodukte aus Pyruvat, dem Endprodukt der Glykolyse. Aspartat wird aus Oxalacetat, einem Zwischenprodukt des Citratzyklus, gebildet. Asparagin, Methionin, Threonin und Lysin werden jeweils durch Umwandlung von Aspartat produziert. Isoleucin wird aus Threonin gebildet. In einem komplexen 9-Schritt-Weg erfolgt die Bildung von Histidin aus 5-Phosphoribosyl-1-pyrophosphat, einem aktivierten Zucker.

Aminosäuren, deren Menge den Proteinbiosynthesebedarf übersteigt, können nicht gespeichert werden, und werden statt dessen abgebaut, so daß Zwischenprodukte für die Haupt-Stoffwechselwege der Zelle bereitgestellt werden (für einen Überblick siehe Stryer, L., Biochemistry, 3. Aufl. Kap. 21 "Amino Acid Degradation and the Urea Cycle"; S 495-516 (1988)). Die Zelle ist zwar in der Lage, ungewünschte Aminosäuren in nützliche Stoffwechsel-Zwischenprodukte umzuwandeln, jedoch ist die Aminosäureproduktion hinsichtlich der Energie, der Vorstufenmoleküle und der für ihre Synthese nötigen Enzyme aufwendig. Es überrascht daher nicht, daß die Aminosäure-Biosynthese durch Feedback-Hemmung reguliert wird, wobei das Vorliegen einer bestimmten Aminosäure ihre eigene Produktion verlangsamt oder ganz beendet (für einen Überblick über Rückkopplungs-Mechanismen bei Aminosäure-Biosynthesewegen, siehe Stryer, L., Biochemistry, 3. Aufl., Kap. 24, "Biosynthesis of Amino Acids and Heme", S. 575-600 (1988)). Der Ausstoß einer bestimmten Aminosäure wird daher durch die Menge dieser Aminosäure in der Zelle eingeschränkt.

### B. Metabolismus und Verwendungen von Vitaminen, Cofaktoren und Nutrazeutika

Vitamine, Cofaktoren und Nutrazeutika umfassen eine weitere Gruppe von Molekülen. Höhere Tiere haben die Fähigkeit verloren, diese zu synthetisieren und müssen sie somit aufnehmen, obwohl sie leicht durch andere Organismen, wie Bakterien, synthetisiert werden. Diese Moleküle sind entweder biologisch aktive Moleküle an sich oder Vorstufen von biologisch aktiven Substanzen, die als Elektronenüberträger oder Zwischenprodukte bei einer Reihe von Stoffwechselwegen dienen. Diese Verbindungen haben neben ihrem Nährwert auch einen signifikanten industriellen Wert als Farbstoffe, Antioxidantien und Katalysatoren oder andere Verarbeitungs-Hilfsstoffe. (Für einen Überblick über die Struktur, Aktivität und die industriellen Anwendungen dieser Verbindungen siehe bspw. Ullman's Encyclopedia of Industrial Chemistry, "Vitamins", Bd. A27, S. 443-613, VCH: Weinheim, 1996). Der Begriff "Vitamin" ist im Fachgebiet bekannt und umfaßt Nährstoffe, die von einem Organismus für eine normale Funktion benötigt werden, jedoch nicht von diesem Organismus selbst synthetisiert werden können. Die Gruppe der Vitamine kann Cofaktoren und nutrazeutische Verbindungen umfassen. Der Begriff "Cofaktor" umfaßt nicht-proteinartige Verbindungen, die für das Auftreten einer normalen Enzymaktivität nötig sind. Diese Verbindungen können organisch oder anorganisch sein; die erfindungsgemäßen Cofaktor-Moleküle sind vorzugsweise organisch. Der Begriff "Nutrazeutikum" umfaßt Nahrungsmittelzusätze, die bei Pflanzen und Tieren, insbesondere dem Menschen, gesundheitsfördernd sind. Beispiele solcher Moleküle sind Vitamine, Antioxidantien und ebenfalls bestimmte Lipide (z.B. mehrfach ungesättigte Fettsäuren).

Die Biosynthese dieser Moleküle in Organismen, die zu ihrer Produktion befähigt sind, wie Bakterien, ist umfassend charakterisiert worden (Ullman's Encyclopedia of Industrial Chemistry, "Vitamins", Bd. A27, S. 443-613, VCH: Weinheim, 1996, Michal, G. (1999) Biochemical Pathways: An Atlas of Biochemistry and Molecular Biology, John Wiley & Sons; Ong, A.S., Niki, E. und Packer, L. (1995) "Nutrition, Lipids, Health and Disease" Proceedings of the UNESCO/Confederation of Scientific and Technological Associations in Malaysia and the Society for free Radical Research - Asien, abgehalten am 1.-3. Sept. 1994 in Penang, Malaysia, AOCS Press, Champaign, IL X, 374 S).

Thiamin (Vitamin B₁) wird durch chemisches Kuppeln von Pyrimidin und Thiazol-Einheiten gebildet. Riboflavin (Vitamin B₂) wird aus Guanosin-5'-triphosphat (GTP) und Ribose-5'-phosphat synthetisiert. Riboflavin wiederum wird zur Synthese von Flavinmononukleotid (FMN) und Flavinadenindinukleotid (FAD) eingesetzt. Die Familie von Verbindungen, die gemeinsam als "Vitamin B₆" bezeichnet werden (bspw. Pyridoxin, Pyridoxamin, Pyridoxal-5'-phosphat und das kommerziell verwendete Pyridoxinhydrochlorid), sind alle Derivate der gemeinsamen Struktureinheit 5-Hydroxy-6-methylpyridin. Panthothenat (Pantothensäure, R-(+)-N-(2,4-Dihydroxy-3,3-dimethyl-1-oxobutyl)-β-alanin) kann entweder durch chemische Synthese oder durch Fermentation hergestellt werden. Die letzten Schritte bei der Pantothenat-Biosynthese bestehen aus der ATP-getriebenen Kondensation von β-Alanin und Pantoinsäure. Die für die Biosyntheseschritte für die Umwandlung in Pantoinsäure, in β-Alanin und zur Kondensation in Pantothensäure verantwortlichen Enzyme sind bekannt. Die metabolisch aktive Form von Pantothenat ist Coenzym A, dessen Biosynthese über 5 enzymatische Schritte verläuft. Pantothenat, Pyridoxal-5'-phosphat, Cystein und ATP sind die Vorstufen von Coenzym A. Diese Enzyme katalysieren nicht nur die Bildung von Pantothenat, sondern auch die Produktion von (R)-Pantoinsäure, (R)-Pantolacton, (R)-Panthenol (Provitamin B₅), Pantethein (und seinen Derivaten) und Coenzym A.

Die Biosynthese von Biotin aus dem Vorstufenmolekül Pimeloyl-CoA in Mikroorganismen ist ausführlich untersucht worden, und man hat mehrere der beteiligten Gene identifiziert. Es hat sich herausgestellt, daß viele der entsprechenden Proteine an der Fe-Cluster-Synthese beteiligt sind und zu der Klasse der nifS-Proteine gehören. Die Liponsäure wird von der Octanonsäure abgeleitet und dient als Coenzym beim Energie-Metabolismus, wo sie Bestandteil des Pyruvatdehydrogenasekomplexes und des α-Ketoglutaratdehydrogenasekomplexes wird. Die Folate sind eine Gruppe von Substanzen, die alle von der Folsäure abgeleitet werden, die wiederum von L-Glutaminsäure, p-Aminobenzoesäure und 6-Methylpterin hergeleitet ist. Die Biosynthese der Folsäure und ihrer Derivate, ausgehend von den Stoffwechselzwischenprodukten Guanosin-5'-triphosphat (GTP), L-Glutaminsäure und p-Aminobenzoesäure ist in bestimmten Mikroorganismen eingehend untersucht worden.

Corrinoide (wie die Cobalamine und insbesondere Vitamin B₁₂) und die Porphyrine gehören zu einer Gruppe von Chemikalien, die sich durch ein Tetrapyrrol-Ringsystem auszeichnen. Die Biosynthese von Vitamin B₁₂ ist hinreichend komplex, daß sie noch nicht vollständig charakterisiert worden ist, jedoch ist inzwischen ein Großteil der beteiligten Enzyme und Substrate bekannt. Nikotinsäure (Nikotinat) und Nikotinamid sind Pyridin-Derivate, die auch als "Niacin" bezeichnet werden. Niacin ist die Vorstufe der wichtigen Coenzyme NAD (Nikotinamidadenindinukleotid) und NADP (Nikotinamidadenindinukleotidphosphat) und ihrer reduzierten Formen.

Die Produktion dieser Verbindungen im Großmaßstab beruht größtenteils auf zellfreien chemischen Synthesen, obwohl einige dieser Chemikalien, wie Riboflavin, Vitamin B₆, Pantothenat und Biotin, auch durch großangelegte Anzucht von Mikroorganismen produziert worden sind. Nur Vitamin B₁₂ wird aufgrund der Komplexität seiner Synthese lediglich durch Fermentation produziert. In-vitro-Verfahren erfordern einen erheblichen Aufwand an Materialien und Zeit und häufig an hohen Kosten.

### C. Purin-, Pyrimidin-, Nukleosid- und Nukleotid-Metabolismus und Verwendungen

Gene für den Purin- und Pyrimidin-Stoffwechsel und ihre entsprechenden Proteine sind wichtige Ziele für die Therapie von Tumorerkrankungen und Virusinfektionen. Der Begriff "Purin" oder "Pyrimidin" umfaßt stickstoffhaltige Basen, die Bestandteile der Nukleinsäuren, Coenzyme und Nukleotide sind. Der Begriff "Nukleotid" beinhaltet die grundlegenden Struktureinheiten der Nukleinsäuremoleküle, die eine stickstoffhaltige Base, einen Pentose-Zucker (bei RNA ist der Zucker Ribose, bei DNA ist der Zucker D-Desoxyribose) und Phosphorsäure umfassen. Der Begriff "Nukleosid" umfaßt Moleküle, die als Vorstufen von Nukleotiden dienen, die aber im Gegensatz zu den Nukleotiden keine Phosphorsäureeinheit aufweisen. Durch Hemmen der Biosynthese dieser Moleküle oder ihrer Mobilisierung zur Bildung von Nukleinsäuremolekülen ist es möglich, die RNA- und DNA-Synthese zu hemmen; wird diese Aktivität zielgerichtet bei Krebszellen gehemmt, läßt sich die Teilungs- und Replikationsfähigkeit von Tumorzellen hemmen. Es gibt zudem Nukleotide, die keine Nukleinsäuremoleküle bilden, jedoch als Energiespeicher (d.h. AMP) oder als Coenzyme (d.h. FAD und NAD) dienen.

Mehrere Veröffentlichungen haben die Verwendung dieser Chemikalien für diese medizinischen Indikationen beschrieben, wobei der Purin- und/oder Pyrimidin-Metabolismus beeinflußt wird (bspw. Christopherson, R.I. und Lyons, S.D. (1990) "Potent inhibitors of de novo pyrimidine and purine biosynthesis as chemotherapeutic agents", Med. Res. Reviews 10:505-548). Untersuchungen an Enzymen, die am Purin- und Pyrimidin-Metabolismus beteiligt sind, haben sich auf die Entwicklung neuer Medikamente konzentriert, die bspw. als Immunsuppressiva oder Antiproliferantien verwendet werden können (Smith, J.L. (1995) "Enzymes in Nucleotide Synthesis" Curr. Opin. Struct. Biol. 5:752-757; (1995) Biochem. Soc. Transact. 23:877-902). Die Purin- und Pyrimidinbasen, Nukleoside und Nukleotide haben jedoch auch andere Einsatzmöglichkeiten: als Zwischenprodukte bei der Biosysnthese verschiedener Feinchemikalien (z.B. Thiamin, S-Adenosyl-methionin, Folate oder Riboflavin), als Energieträger für die Zelle (bspw. ATP oder GTP) und für Chemikalien selbst, die gewöhnlich als Geschmacksverstärker (bspw. IMP oder GMP) oder für viele medizinische Anwendungen verwendet werden (siehe bspw. Kuninaka, A., (1996) "Nucleotides and Related Compounds in Biotechnology Bd. 6, Rehm et al., Hrsg. VCH: Weinheim, S. 561-612). Enzyme, die am Purin-, Pyrimidin-, Nukleosid- oder Nukleotid-Metabolismus beteiligt sind, dienen auch immer stärker als Ziele, gegen die Chemikalien für den Pflanzenschutz, einschließlich Fungiziden, Herbiziden und Insektiziden, entwickelt werden.

Der Metabolismus dieser Verbindungen in Bakterien ist charakterisiert worden (für Übersichten siehe bspw. Zalkin, H. und Dixon, J.E. (1992) "De novo purin nucleotide biosynthesis" in Progress in Nucleic Acids Research and Molecular Biology, Bd. 42, Academic Press, S. 259-287; und Michal, G. (1999) "Nucleotides and Nucleosides"; Kap. 8 in: Biochemical Pathways: An Atlas of Biochemistry and Molecular Biology, Wiley, New York). Der Purin-Metabolismus, das Objekt intensiver Forschung, ist für das normale Funktionieren der Zelle essentiell. Ein gestörter Purin-Metabolismus in höheren Tieren kann schwere Erkrankungen verursachen, bspw. Gicht. Die Purinnukleotide werden aus Ribose-5-phosphat über eine Reihe von Schritten über die Zwischenverbindung Inosin-5'-phosphat (IMP) synthetisiert, was zur Produktion von Guanosin-5'-monophosphat (GMP) oder Adenosin-5'-monophosphat (AMP) führt, aus denen sich die als Nukleotide verwendeten Triphosphatformen leicht herstellen lassen. Diese Verbindungen werden auch als Energiespeicher verwendet, so daß ihr Abbau Energie für viele verschiedene biochemische Prozesse in der Zelle liefert. Die Pyrimidinbiosynthese erfolgt über die Bildung von Uridin-5'-monophosphat (UMP) aus Ribose-5-phosphat. UMP wiederum wird in Cytidin-5'-triphosphat (CTP) umgewandelt. Die Desoxyformen sämtlicher Nukleotide werden in einer Einschritt-Reduktionsreaktion aus der Diphosphat-Riboseform des Nukleotides zur Diphosphat-Desoxyriboseform des Nukleotides hergestellt. Nach der Phosphorylierung können diese Moleküle an der DNA-Synthese teilnehmen.

### D. Trehalose-Metabolismus und Verwendungen

Trehalose besteht aus zwei Glucosemolekülen, die über eine α,α-1,1-Bindung miteinander verknüpft sind. Sie wird gewöhnlich in der Nahrungsmittelindustrie als Süßstoff, als Additiv für getrocknete oder gefrorene Nahrungsmittel sowie in Getränken verwendet. Sie wird jedoch auch in der pharmazeutischen Industrie, der Kosmetik- und Biotechnologie-Industrie angewendet (s. bspw. Nishimoto et al., (1998) US-Patent Nr. 5 759 610; Singer, M.A. und Lindquist, S. (1998) Trends Biotech. 16:460-467; Paiva, C.L.A. und Panek, A.D. (1996) Biotech Ann. Rev. 2:293-314; und Shiosaka, M. (1997) J. Japan 172:97-102). Trehalose wird durch Enzyme von vielen Mikroorganismen produziert und auf natürliche Weise in das umgebende Medium abgegeben, aus dem sie durch im Fachgebiet bekannte Verfahren gewonnen werden kann.

### II. Verwendung und oxidative Phosphorylierung von Zucker und Kohlenstoffmolekülen

Kohlenstoff ist eine entscheidend wichtige Substanz für die Bildung aller organischen Verbindungen und muß somit nicht nur für das Wachstum und die Teilung von *C*. *glutamicum,* sondern auch zur Überproduktion von Feinchemikalien von diesem Mikroorganismus mit der Nahrung aufgenommen werden. Zucker, wie Mono-, Di- oder Polysaccharide, sind besonders gute Kohlenstoffquellen, und somit enthalten Standard-Wachstumsmedien üblicherweise einen oder mehrere aus: Glucose, Fructose, Mannose, Galactose, Ribose, Sorbose, Ribulose, Lactose, Maltose, Saccharose, Raffinose, Stärke oder Zellulose (Ullmann's Encyclopedia of Industrial Chemistry (1987) Bd. A9, "Enzymes", VCH: Weinheim). Alternativ Man können komplexere Zuckerverbindungen, wie Melassen oder Nebenprodukte der Zukker-Raffinierung, in den Medien verwendet werden. Andere Verbindungen neben Zuckern können als alternative Kohlenstoffquellen verwendet werden, wie z.B. Alkohole (z.B. Ethanol oder Methanol), Alkane, Zuckeralkohole, Fettsäuren und organische Säuren (z.B. Essigsäure oder Milchsäure). Einen Überblick über die Kohlenstoffquellen und ihre Nutzung durch Mikroorganismen in Kultur s. in Ullman's Encyclopedia of Industrial Chemistry (1987) Bd. A9, "Enzymes", VCH: Weinheim; Stoppok, E., und Buchholz, K. (1996) "Sugar-based raw materials for fermentation applications" in Biotechnology (Rehm, H.J., et al., Hrsg.) Bd. 6, VCH:Weinheim, S. 5-29; Rehm, H.J. (1980) Industrielle Mikrobiologie, Springer: Berlin; Batholomew, W.H., und Reiman, H.B. (1979) Economics of Fermentation Processes, in Peppler, H.J., und Perlman, D., Hrsg., Microbial Technology, 2. Aufl., Bd. 2, Kapitel 18, Academic Press: New York; und Kockova-Kratachvilowa, A. (1981), Bd. 1, Kapitel 1, Verlag Chemie: Weinheim.

Nach der Aufnahme müssen diese energiereichen Kohlenstoffmoleküle verarbeitet werden, so daß sie durch einen der hauptsächlichen Zuckerstoffwechselwege abgebaut werden können. Diese Wege führen direkt zu nützlichen Abbauprodukten, wie Ribose-5-phosphat und Phosphoenolpyruvat, das anschließend in Pyruvat umgewandelt werden kann. Die drei wichtigsten Wege für den Zuckerstoffwechsel in Bakterien sind u. a. der Embden-Meyerhoff-Parnas- (EMP-) Weg (auch als Glykolyse oder Fructosebisphosphat-Weg bekannt), der Hexosemonophosphat- (HMP-) Weg (auch als Pentose-Nebenstoffwechselweg oder Pentosephosphat-Weg bekannt) und der Entner-Doudoroff- (ED-) Weg (eine Übersicht s. in Michal, G. (1999) Biochemical Pathways: An Atlas of Biochemistry and Molecular Biology, Wiley: New York, und Stryer, L. (1988) Biochemistry, Kapitel 13-19, Freeman: New York, und darin zitierte Literaturstellen).

Der EMP-Weg wandelt Hexose-Moleküle in Pyruvat um, und der Prozeß erzeugt 2 Moleküle ATP und 2 Moleküle NADH. Ausgehend von Glucose-1-phosphat (das entweder direkt mit dem Medium aufgenommen oder alternativ aus Glykogen, Stärke oder Zellulose erzeugt werden kann) wird das Glucosemolekül zu Fructose-6-phosphat isomerisiert, phosphorylie und in zwei Moleküle Glycerinaldehyd-3-phosphat mit je 3 Kohlenstoffatomen gespalten. Nach dem Dehydrieren, Phosphorylieren und nachfolgenden Umlagerungen entsteht Pyruvat.

Der HMP-Weg wandelt Glucose in Reduktionsäquivalente, wie NADH, um und erzeugt Pentose- und Tetroseverbindungen, die als Zwischenprodukte und Vorstufen bei einer Reihe anderer Stoffwechselwege benötigt werden. Im HMP-Weg wird Glucose-6-phosphat durch zwei aufeinanderfolgende Dehydrogenase-Reaktionen (die auch zwei NADPH-Moleküle freisetzen) und einen Carboxylierungsschritt in Ribulose-5-phosphat umgewandelt. Ribulose-5-phosphat kann auch in Xylulose-5-phosphat und Ribose-5-phosphat umgewandelt werden; ersteres kann in einer Reihe biochemischer Schritte zu Glucose-6-phosphat werden, das in den EMP-Weg eintreten kann, wohingegen letzteres gewöhnlich als Zwischenprodukt in anderen Biosynthesewegen in der Zelle verwendet wird.

Der ED-Weg beginnt mit der Verbindung Glucose oder Gluconat, die anschließend phosphoryliert und dehydratisiert wird, so daß 2-Dehydro-3-desoxy-6-P-gluconat entsteht. Auch Glucuronat und Galacturonat können über komplexere biochemische Wege in 2-Dehydro-3-desoxy-6-P-gluconat umgewandelt werden. Dieses Produktmolekül wird anschließend in Glycerinaldehyd-3-P und Pyruvat gespalten; Glycerinaldehyd-3-P kann selbst in Pyruvat umgewandelt werden.

Der EMP- und der HMP-Weg haben viele gleiche Merkmale, einschließlich der Zwischenprodukte und Enzyme. Der EMP-Weg liefert die größte Menge an ATP, erzeugt aber weder Ribose-5-phosphat, eine wichtige Vorstufe für beispielsweise die Nukleinsäurebiosynthese, noch erzeugt er Erythrose-4-phosphat, das für die Aminosäurebiosynthese wichtig ist. Mikroorganismen, die nur den EMP-Weg zur Glucosenutzung verwenden können, können somit nicht auf einfachen Medien mit Glucose als einziger Kohlenstoffquelle wachsen. Sie werden als anspruchsvolle Organismen bezeichnet, und ihr Wachstum erfordert die Zufuhr komplexer organischer Verbindungen, wie man sie im Hefeextrakt findet.

Der HMP-Weg produziert dagegen alle Vorstufen, die für die Nukleinsäure- und Aminosäurebiosynthese nötig sind, liefert aber nur die Hälfte der Menge an ATP-Energie, die der EMP-Weg liefert. Der HMP-Weg produziert auch NADPH, das für Redox-Reaktionen in Biosynthesewegen verwendet werden kann. Der HMP-Weg erzeugt jedoch nicht direkt Pyruvat, und folglich müssen diese Mikroorganismen auch einen Teil des EMP-Weges besitzen. Daher überrascht es nicht, daß eine Reihe von Mikroorganismen sich im Verlauf der Evolution so entwickelt haben, daß sie beide Wege besitzen.

Die durch diese Wege erzeugten Pyruvatmoleküle können leicht über den Krebs-Zyklus (auch als Citronensäurezyklus, Citratzyklus oder Tricarbonsäurezyklus (TCA-Zyklus) bekannt) in Energie umgewandelt werden. Bei diesem Prozeß wird Pyruvat zunächst decarboxyliert, was zur Produktion von einem Molekül NADH, 1 Molekül Acetyl-CoA und einem Molekül CO₂ führt. Dann reagiert die Acetylgruppe von Acetyl-CoA mit dem aus 4 Kohlenstoffatomen bestehenden Oxalacetat, was zur Bildung von Citronensäure, eine organischen Säure mit 6 Kohlenstoffatomen führt. Schließlich wird Oxalacetat regeneriert und kann wiederum als Acetylakzeptor dienen, wodurch der Zyklus vervollständigt wird. Die während der Oxidation von Zwischenprodukten im TCA-Zyklus freigesetzten Elektronen werden auf NAD⁺ übertragen, was NADH ergibt.

Während der Atmung werden die Elektronen von NADH auf molekularen Sauerstoff oder andere terminale Elektronenakzeptoren übertragen. Dieser Prozeß wird von der Atmungskette katalysiert, einem Elektronentransportsystem, das sowohl integrale Membranproteine als auch membranassoziierte Proteine enthält. Dieses System hat zwei grundlegende Aufgaben: erstens akzeptiert es Elektronen von einem Elektronendonor und überträgt sie an einen Elektronenakzeptor und zweitens konserviert es einen Teil der Energie, die während des Elektronentransfers freigesetzt wird, durch Synthese von ATP. Es ist bekannt, daß mehrere Typen von Oxidations-Reduktions-Enzymen und Elektronentransportproteinen an solchen Prozessen beteiligt sind, einschließlich der NADH-Dehydrogenasen, flavinhaltigen Elektronenüberträger, Eisen-Schwefel-Proteine und Cytochrome. Die NADH-Dehydrogenasen befinden sich an der cytosolischen Oberfläche der Plasmamembran und übertragen Wasserstoffatome von NADH auf Flavoproteine, die wiederum Elektronen von NADH akzeptieren. Die Flavoproteine sind eine Gruppe von Elektronenüberträgern, die eine prosthetische Flavingruppe besitzen, die alternativ reduziert und oxidiert wird, wenn sie Elektronen akzeptiert und überträgt. Bekanntlich nehmen drei Flavine an diesen Reaktionen teil: Riboflavin, Flavinadenindinukleotid (FAD) und Flavinmononukleotid (FMN). Eisen-Schwefel-Proteine enthalten ein Cluster von Eisen- und Schwefelatomen, die nicht an eine Häm-Gruppe gebunden sind, aber trotzdem an Dehydratisierungs- und Rehydratisierungsreaktionen teilnehmen können. Succinatdehydrogenase und Aconitase sind Beispiele für Eisen-Schwefel-Proteine; ihre Eisen-Schwefel-Komplexe können Elektronen als Teil der gesamten Elektronentransportkette akzeptieren und übertragen. Die Cytochrome sind Proteine, die einen Eisen-Porphyrinring (Häm) enthalten. Es gibt eine Reihe verschiedener Klassen von Cytochromen, die sich in ihren Reduktionspotentialen unterscheiden. Funktionelle bilden diese Cytochrome Wege, in denen Elektronen an andere Cytochrome übertragen werden können, die immer positivere Reduktionspotentiale besitzen. Eine weitere Klasse von Nicht-Protein-Elektronenüberträgern ist bekannt: die lipidlöslichen Chinone (z.B. Coenzym Q). Diese Moleküle dienen auch als Wasserstoffatom-Akzeptoren und Elektronendonoren.

Die Wirkung der Atmungskette erzeugt einen Protonengradienten über die Zellmembran, was die protonenmotorische Kraft hervorruft. Diese Kraft wird von der Zelle zur ATP-Synthese über das membranspannende Enzym ATP-Synthase ausgenutzt. Dieses Enzym ist ein Multiproteinkomplex, bei dem der Transport von H⁺-Molekülen durch die Membran zur physikalischen Rotation der intrazellulären Untereinheiten und zur gleichzeitigen Phosphorylierung von ADT unter Bildung von ATP führt (s. die Übersicht in Fillingame, R.H., und Divall, S. (1999) Novartis Found Symp. 221:218-229, 229-234).

Nicht-Hexose-Kohlenstoffsubstrate können ebenfalls als Kohlenstoff- und Energiequelle für Zellen dienen. Diese Substrate können zunächst im Gluconeogenese-Weg in Hexosezucker umgewandelt werden, wobei die Glucose zuerst von der Zelle synthetisiert und dann zur Energieerzeugung abgebaut wird. Das Ausgangsmaterial für diese Reaktion ist Phosphoenolpyruvat (PEP), eines der wichtigsten Zwischenprodukte der Glykolyse. PEP kann anstatt aus Zuckern auch aus anderen Substraten, wie Essigsäure, oder durch Decarboxylierung von Oxalacetat (das selbst Zwischenprodukt des TCA-Zyklus ist) gebildet werden. Durch Umkehrung der Glykolyse (unter Verwendung einer anderen Enzymkaskade als bei der ursprünglichen Glykolyse) kann Glucose-6-phosphat gebildet werden. Die Umwandlung von Pyruvat in Glucose erfordert die Verwendung von 6 energiereichen Phosphatbindungen, wogegen die Glykolyse bei der Umwandlung von Glucose in Pyruvat nur 2 ATP erzeugt. Die vollständige Oxidation von Glucose (Glykolyse, Umwandlung von Pyruvat in Acetyl-CoA, Citronensäurezyklus und oxidative Phosphorylierung) ergibt zwischen 36-38 ATP, so daß dem Nettoverlust an energiereichen Phosphatbindungen während der Gluconeogenese ein insgesamt höherer Gewinn an diesen energiereichen Molekülen, die durch die Oxidation von Glucose erzeugt werden, gegenübersteht.

### III. Erfindungsgemäße Elemente und Verfahren

Die vorliegende Erfindung beruht zumindest teilweise auf der Entdeckung von neuen Molekülen, die hier als SMP-Nukleinsäure- und -Protein-Moleküle bezeichnet werden und an der Umwandlung von Zuckern in nützliche Abbauprodukte und Energie (z.B. Energie) in *C. glutamicum* teilnehmen oder an der Produktion nützlicher energiereicher Moleküle (z.B. ATP) durch andere Prozesse, wie oxidative Phosphorylierung, teilnehmen können. Bei einer Ausführungsform nehmen die SMP-Moleküle am Stoffwechsel von Kohlenstoffverbindungen, wie Zuckern, oder an der Erzeugung von Energiemolekülen (z.B. ATP) durch Prozesse, wie oxidative Phosphorylierung, in *Corynebacterium glutamicum* teil. Die Aktivität der erfindungsgemäßen SMP-Moleküle, zum Kohlenstoffmetabolismus und zur Energieproduktion in *C. glutamicum* beizutragen, hat bei einer bevorzugten Ausführungsform eine Auswirkung auf die Produktion einer gewünschten Feinchemikalie durch diesen Organismus. Bei einer besonders bevorzugten Ausführungsform wird die Aktivität der erfindungsgemäßen SMP-Moleküle moduliert, so daß die Stoffwechsel- und Energiewege von *C*. *glutamicum,* an denen die erfindungsgemäßen SMP-Proteine teilnehmen, hinsichtlich der Ausbeute, Produktion und/oder Effizienz der Produktion moduliert werden, die entweder direkt oder indirekt die Ausbeute, Produktion und/oder Effizienz der Produktion einer gewünschten Feinchemikalie aus *C*. *glutamicum* modulieren.

Der Begriff "SMP-Protein" oder "SMP-Polypeptid" umfaßt Proteine, die eine am Stoffwechsel von Kohlenstoffverbindungen, wie Zukkern, und an der Erzeugung von Energiemolekülen (z.B. ATP) durch Prozesse, wie oxidative Phosphorylierung, beteiligte Funktion in *Corynebacterium glutamicum* ausüben können. Beispiele für SMP-Proteine umfassen solche, die von den in Tabelle 1 und Anhang A aufgeführten SMP-Genen codiert werden. Die Ausdrücke "SMP-Gen" oder *"*SMP-Nukleinsäuresequenz*"* umfassen Nukleinsäuresequenzen, die ein SMP-Protein codieren, das aus einem codierenden Bereich und entsprechenden untranslatierten 5'- und 3'-Sequenzbereichen besteht. Beispiele für SMP-Gene sind die in Tabelle 1 aufgelisteten. Die Begriffe "Produktion" oder "Produktivität" sind im Fachgebiet bekannt und beinhalten die Konzentration des Fermentationsproduktes (bspw. der gewünschten Feinchemikalie), das innerhalb einer festgelegten Zeitspanne und eines festgelegten Fermentationsvolumens gebildet wird (bspw. kg Produkt pro Std. pro 1). Der Begriff "Effizienz der Produktion" umfaßt die Zeit, die zur Erzielung einer bestimmten Produktionsmenge nötig ist (bspw. wie lange die Zelle zur Aufrichtung einer bestimmten Ausstoßrate einer Feinchemikalie benötigt). Der Begriff "Ausbeute" oder "Produkt/Kohlenstoff-Ausbeute" ist im Fachgebiet bekannt und umfaßt die Effizienz der Umwandlung der Kohlenstoffquelle in das Produkt (d.h. die Feinchemikalie). Dies wird bspw. gewöhnlich ausgedrückt als kg Produkt pro kg Kohlenstoffquelle. Durch Vergrößern der Ausbeute oder Produktion der Verbindung wird die Menge der gewonnenen Moleküle oder der geeigneten gewonnenen Moleküle dieser Verbindung in einer bestimmten Kulturmenge über einen festgelegten Zeitraum erhöht. Die Begriffe "Biosynthese" oder "Biosyntheseweg" sind im Fachgebiet bekannt und umfassen die Synthese einer Verbindung, vorzugsweise einer organischen Verbindung, durch eine Zelle aus Zwischenverbindungen, bspw. in einem Mehrschritt- oder stark regulierten Prozeß. Die Begriffe "Abbau" oder "Abbauweg" sind im Fachgebiet bekannt und umfassen die Spaltung einer Verbindung, vorzugsweise einer organischen Verbindung, durch eine Zelle in Abbauprodukte (allgemeiner gesagt, kleinere oder weniger komplexe Moleküle), bspw. in einem Mehrschritt- oder stark regulierten Prozeß. Der Ausdruck "Abbauprodukt" ist im Fachgebiet bekannt und beinhaltet Abbauprodukte einer Verbindung. Diese Produkte können selbst als Vorstufen (Ausgangspunkt) oder Zwischenmoleküle geeignet sein, die für die Biosynthese anderer Verbindungen durch die Zelle notwendig sind. Der Begriff "Metabolismus" ist im Fachgebiet bekannt und umfaßt die Gesamtheit der biochemischen Reaktionen, die in einem Organismus stattfinden. Der Metabolismus einer bestimmten Verbindung (z.B. der Metabolismus einer Aminosäure, wie Glycin) umfaßt dann sämtliche Biosynthese-, Modifikations- und Abbauwege in der Zelle, die diese Verbindung betreffen.

Die erfindungsgemäßen SMP-Moleküle sind in einer anderen Ausführungsform befähigt, die Produktion eines gewünschten Moleküls, wie einer Aminosäure, in einem Mikroorganismus, wie *C*. *glutamicum*, zu modulieren. Es gibt eine Reihe von Mechanismen, durch die die Änderung eines erfindungsgemäßen SMP-Proteins die Ausbeute, Produktion und/oder Effizienz der Produktion einer Feinchemikalie aus einem *C. glutamicum*-Stamm, der ein solches verändertes Protein enthält, direkt beeinflussen kann. Der Abbau von energiereichen Kohlenstoffmolekülen, wie Zuckern, und die Umwandlung von Verbindungen, wie NADH und FADH₂ über die oxidative Phosphorylierung in nützlichere Verbindungen führt zu einer Reihe von Verbindungen, die selbst wünschenswerte Feinchemikalien sein können, wie Pyruvat, ATP, NADH und eine Reihe von Zucker-Zwischenverbindungen. Ferner werden die Energiemoleküle (wie ATP) und die Reduktionsäquivalente (wie NADH oder NADPH), die durch diese Stoffwechselwege produziert werden, in der Zelle zum Antreiben von Reaktionen verwendet, die ansonsten energetisch ungünstig wären. Zu solchen ungünstigen Reaktionen gehören viele Biosynthesewege von Feinchemikalien. Durch die Verbesserung der Fähigkeit der Zelle, einen bestimmten Zucker zu nutzen (z.B. durch Manipulation der Gene, die am Abbau und an der Umwandlung des Zuckers in Energie für die Zelle beteiligt sind), läßt sich die Menge an Energie, die verfügbar ist, damit ungünstige und trotzdem gewünschte Stoffwechselreaktionen (z.B. die Biosynthese einer gewünschten Feinchemikalie) auftreten, vergrößern.

Die Mutagenese von einem oder mehreren erfindungsgemäßen SMP-Proteinen kann auch zu SMP-Proteinen mit geänderten Aktivitäten führen, die indirekt die Produktion einer oder mehrerer gewünschten Feinchemikalien aus *C*. *glutamicum* beeinflussen. Beispielsweise kann man durch Erhöhen der Effizienz der Nutzung eines oder mehrerer Zucker (so daß die Umwandlung des Zuckers in nutzbare Energiemoleküle verbessert ist) oder durch Erhöhen der Effizienz der Umwandlung von Reduktionsäquivalenten in nutzbare Energiemoleküle (z.B. durch Verbesserung der Effizienz der oxidativen Phosphorylierung oder der Aktivität der ATP-Synthase) die Menge dieser energiereichen Verbindungen, die für die Zelle zum Antrieb normalerweise ungünstiger Stoffwechselprozesse verfügbar ist, steigern. Zu diesen Prozessen gehört der Aufbau der Zellwände, die Transkription, Translation, die Biosynthese von Verbindungen, die für das Wachstum und die Teilung von Zellen nötig sind (z.B. Nukleotide, Aminosäuren, Vitamine, Lipide usw.) (Lengeler et al. (1999) Biology of Prokaryotes, Thieme Verlag: Stuttgart, S. 88-109; 913-918; 875-899). Durch Verbessern von Wachstum und Vermehrung dieser veränderten Zellen ist es möglich, die Lebensfähigkeit der Zellen in Kulturen im Großmaßstab zu steigern und auch ihre Teilungsrate zu verbessern, so daß eine vergleichsweise größere Anzahl Zellen in der Fermenterkultur überleben kann. Die Ausbeute, Produktion oder Effizienz der Produktion kann zumindest aufgrund der Anwesenheit einer größeren Anzahl lebensfähiger Zellen, die jeweils die gewünschte Feinchemikalie produzieren, erhöht werden. Auch viele der Abbau- und Zwischenverbindungen, die während des Zuckerstoffwechsels produziert werden, sind notwendige Vorstufen oder Zwischenprodukte für andere Biosynthesewege in der Zelle. Beispielsweise werden viele Aminosäuren direkt aus Verbindungen synthetisiert, die normalerweise aus der Glykolyse oder dem TCA-Zyklus hervorgehen (z.B. wird Serin aus 3-Phosphoglycerat, einem Zwischenprodukt der Glykolyse, synthetisiert). So ist es durch Steigerung der Effizienz der Umwandlung von Zuckern in nützliche Energiemoleküle auch möglich, die Menge an nützlichen Abbauprodukten zu steigern.

Als Ausgangspunkt zur Herstellung der erfindungsgemäßen Nukleinsäuresequenzen eignet sich das Genom eines *Corynebacterium glutamicum*-Stammes, der von der American Type Culture Collection unter der Bezeichnung ATCC 13032 erhältlich ist.

Von diesen Nukleinsäuresequenzen lassen sich durch die in Tabelle 1 bezeichneten Veränderungen die erfindungsgemäßen Nukleinsäuresequenzen mit üblichen Verfahren herstellen.

Ein erfindungsgemäßes SMP-Protein oder ein biologisch aktiver Abschnitt kann am Stoffwechsel von Kohlenstoffverbindungen, wie Zuckern, oder an der Erzeugung von Energiemolekülen (z.B. ATP) durch Prozesse, wie oxidative Phosphorylierung, in *Corynebacterium glutamicum* beteiligt sein oder kann eine oder mehrere der in Tabelle 1 aufgeführten Aktivitäten aufweisen.

In den nachstehenden Unterabschnitten sind verschiedene Aspekte der Erfindung ausführlicher beschrieben:

### A. Isolierte Nukleinsäuremoleküle

Ein Aspekt der Erfindung betrifft isolierte Nukleinsäuremoleküle, die SMP-Moleküle oder biologisch aktive Abschnitte davon codieren, sowie Nukleinsäurefragmente, die zur Verwendung als Hybridisierungssonden oder Primer zur Identifizierung oder Amplifizierung von SMP-codierenden Nukleinsäuren (z.B. SMP-DNA) hinreichen. Der Begriff "Nukleinsäuremolekül", wie hier verwendet, soll DNA-Moleküle (z.B. cDNA oder genomische DNA) und RNA-Moleküle (z.B. mRNA) sowie DNA- oder RNA-Analoga, die mittels Nukleotidanaloga erzeugt werden, umfassen. Dieser Begriff umfaßt zudem die am 3'- und am 5'-Ende des codierenden Genbereichs gelegene untranslatierte Sequenz: mindestens etwa 100 Nukleotide der Sequenz stromaufwärts des 5'-Endes des codierenden Bereichs und mindestens etwa 20 Nukleotide der Sequenz stromabwärts des 3'-Endes des codierenden Bereichs des Gens. Das Nukleinsäuremolekül kann einzelsträngig oder doppelsträngig sein, ist aber vorzugsweise doppelsträngige DNA. Ein "isoliertes" Nukleinsäuremolekül wird von anderen Nukleinsäuremolekülen abgetrennt, die in der natürlichen Quelle der Nukleinsäure zugegen sind. Eine "isolierte" Nukleinsäure hat vorzugsweise keine Sequenzen, die die Nukleinsäure in der genomischen DNA des Organismus, aus dem die Nukleinsäure stammt, natürlicherweise flankieren (bspw. Sequenzen, die sich am 5'- bzw. 3'-Ende der Nukleinsäure befinden). In verschiedenen Ausführungsformen kann bspw. das isolierte SMP-Nukleinsäuremolekül weniger als etwa 5 kb, 4 kb, 3 kb, 2 kb, 1 kb, 0,5 kb oder 0,1 kb der Nukleotidsequenzen, die natürlicherweise das Nukleinsäuremolekül in der genomischen DNA der Zelle, aus der die Nukleinsäure stammt (bspw. eine *C*. *glutamicum*-Zelle) flankieren. Ein "isoliertes" Nukleinsäuremolekül, wie ein cDNA-Molekül, kann überdies im wesentlichen frei von anderem zellulären Material oder Kulturmedium sein, wenn es durch rekombinante Techniken hergestellt wird, oder frei von chemischen Vorstufen oder anderen Chemikalien sein, wenn es chemisch synthetisiert wird.

Ein erfindungsgemäßes Nukleinsäuremolekül, bspw. eine Nukleinsäuremolekül mit einer Nukleotidsequenz aus Anhang A oder ein Abschnitt davon, kann mittels molekularbiologischer Standard-Techniken und der hier bereitgestellten Sequenzinformation hergestellt werden. Bspw. kann eine *C*. *glutamicum*-SMP-cDNA aus einer *C*. *glutamicum*-Bank isoliert werden, indem eine vollständige Sequenz aus Anhang A oder ein Abschnitt davon als Hybridisierungssonde und Standard-Hybridisierungstechniken (wie bspw. beschrieben in Sambrook, J., Fritsch, E.F. und Maniatis, T. Molecular Cloning: A Laboratory Manual. 2. Aufl., Cold Spring Harbor Laboratory, Cold Spring Harbor Laboratory Press, Cold Spring Harbor, NY, 1989) verwendet werden. Überdies läßt sich ein Nukleinsäuremolekül, umfassend eine vollständige Sequenz aus Anhang A oder einen Abschnitt davon, durch Polymerasekettenreaktion isolieren, wobei die Oligonukleotidprimer, die auf der Basis dieser Sequenz erstellt wurden, verwendet werden (z.B. kann ein Nukleinsäuremolekül, umfassend eine vollständige Sequenz aus Anhang A oder einen Abschnitt davon, durch Polymerasekettenreaktion isoliert werden, indem Oligonukleotidprimer verwendet werden, die auf der Basis dieser gleichen Sequenz aus Anhang A erstellt worden sind). Bspw. läßt sich mRNA aus normalen Endothelzellen isolieren (bspw. durch das Guanidiniumthiocyanat-Extraktionsverfahren von Chirgwin et al. (1979) Biochemistry 18: 5294-5299), und die cDNA kann mittels reverser Transkriptase (bspw. Moloney-MLV-Reverse-Transkriptase, erhältlich bei Gibco/BRL, Bethesda, MD, oder AMV-Reverse-Transkriptase, erhältlich von Seikagaku America, Inc., St. Petersburg, FL) hergestellt werden. Synthetische Oligonukleotidprimer für die Amplifizierung via Polymerasekettenreaktion lassen sich auf der Basis einer der in Anhang A gezeigten Nukleotidsequenzen erstellen. Eine erfindungsgemäße Nukleinsäure kann mittels cDNA oder alternativ genomischer DNA als Matrize und geeigneten Oligonukleotidprimern gemäß PCR-Standard-Amplifikationstechniken amplifiziert werden. Die so amplifizierte Nukleinsäure kann in einen geeigneten Vektor kloniert werden und durch DNA-Sequenzanalyse charakterisiert werden. Oligonukleotide, die einer SMP-Nukleotidsequenz entsprechen, können ferner durch Standard-Syntheseverfahren, bspw. mit einem automatischen DNA-Synthesegerät, hergestellt werden.

Bei einer bevorzugten Ausführungsform umfaßt ein erfindungsgemäßes isoliertes Nukleinsäuremolekül eine der in Anhang A aufgeführten Nukleotidsequenzen.

Bei einer weiteren bevorzugten Ausführungsform umfaßt ein erfindungsgemäßes isoliertes Nukleinsäuremolekül ein zu einer der in Anhang A gezeigten Nukleotidsequenzen komplementäres Nukleinsäuremolekül oder einen Abschnitt davon, wobei es sich um ein Nukleinsäuremolekül handelt, das zu einer der in Anhang A gezeigten Nukleotidsequenzen hinreichend komplementär ist, daß es mit einer der in Anhang A angegebenen Sequenzen hybridisieren kann, wodurch ein stabiler Duplex entsteht.

Bei einer Ausführungsform codiert das erfindungsgemäße Nukleinsäuremolekül ein Protein oder einen Abschnitt davon, der eine Aminosäuresequenz umfaßt, die hinreichend homolog zu einer Aminosäuresequenz von Anhang B ist, daß das Protein oder ein Abschnitt davon die Fähigkeit behält, eine am Stoffwechsel von Kohlenstoffverbindungen, wie Zuckern, oder an der Erzeugung von Energiemolekülen (z.B. ATP) durch Prozesse, wie oxidative Phosphorylierung, beteiligte Funktion in *Corynebacterium glutamicum* auszuüben. Wie hier verwendet, betrifft der Begriff "hinreichend homolog" Proteine oder Abschnitte davon, deren Aminosäuresequenzen eine minimale Anzahl identischer oder äquivalenter (bspw. einen Aminosäurerest mit einer ähnlichen Seitenkette wie ein Aminosäurerest in einer der Sequenzen von Anhang B) Aminosäurereste zu einer Aminosäuresequenz aus Anhang B aufweisen, so daß das Protein oder ein Abschnitt davon eine am Stoffwechsel von Kohlenstoffverbindungen, wie Zuckern, oder an der Erzeugung von Energiemolekülen (z.B. ATP) durch Prozesse, wie oxidative Phosphorylierung, beteiligte Funktion in *Corynebacterium glutamicum* ausüben kann. Proteinbestandteile dieser Zucker-Stoffwechselwege oder Energieproduktionssysteme, wie hier beschrieben, können eine Rolle bei der Produktion und Sekretion einer oder mehrerer Feinchemikalien spielen. Beispiele dieser Aktivitäten sind ebenfalls hier beschrieben. Somit trägt die "Funktion eines SMP-Proteins" entweder direkt oder indirekt zur Ausbeute, Produktion und/oder Effizienz der Produktion einer oder mehrerer Feinchemikalien bei. In Tabelle 1 sind Beispiele der SMP-Proteinaktivitäten angegeben.

Abschnitte von Proteinen, die von den erfindungsgemäßen SMP-Nukleinsäuremolekülen codiert werden, sind vorzugsweise biologisch aktive Abschnitte von einem der SMP-Proteine. Der Begriff "biologisch aktiver Abschnitt eines SMP-Proteins", wie er hier verwendet wird, soll einen Abschnitt, bspw. eine Domäne/ein Motiv eines SMP-Proteins, umfassen, die/das am Stoffwechsel von Kohlenstoffverbindungen, wie Zuckern, oder an Energierezugungswegen in *C*. *glutamicum* beteiligt ist oder eine in Tabelle 1 angegebene Aktivität aufweist. Zur Bestimmung, ob ein SMP-Protein oder ein biologisch aktiver Abschnitt davon am Transport am Stoffwechsel von Kohlenstoffverbindungen oder an der Erzeugung von energiereicher Moleküle in *C*. *glutamicum* beteiligt sein kann, kann ein Test der enzymatischen Aktivität durchgeführt werden. Diese Testverfahren, wie eingehend beschrieben in Beispiel 8 des Beispielteils, sind dem Fachmann geläufig.

Zusätzlich zu natürlich vorkommenden Varianten der SMP-Sequenz, die in der Population existieren können, ist der Fachmann sich ebenfalls bewußt darüber, daß Änderungen durch Mutation in eine Nukleotidsequenz von Anhang A eingebracht werden können, was zur Änderung der Aminosäuresequenz des codierten SMP-Proteins führt, ohne daß die Funktionsfähigkeit des SMP-Proteins beeinträchtigt wird. Bspw. lassen sich Nukleotidsusbtitutionen, die an "nicht-essentiellen" Aminosäureresten zu Aminosäuresubstitutionen führen, in einer Sequenz von Anhang A herstellen. Ein "nicht-essentieller" Aminosäurerest ist ein Rest, der sich in der Wildtypsequenz von einem der SMP-Proteine (Anhang B) verändern läßt, ohne daß die Aktivität des SMP-Proteins verändert wird, wohingegen ein "essentieller" Aminosäurerest für die SMP-Proteinaktivität erforderlich ist. Andere Aminosäurereste jedoch (bspw. nicht-konservierte oder lediglich semikonservierte Aminosäurereste in der Domäne mit SMP-Aktivität) können für die Aktivität nicht essentiell sein und lassen sich somit wahrscheinlich verändern, ohne daß die SMP-Aktivität verändert wird.

Ein isoliertes Nukleinsäuremolekül, das ein SMP-Protein codiert, das zu einer Proteinsequenz aus Anhang B homolog ist, kann durch Einbringen von einer oder mehreren Nukleotidsubstitutionen, -additionen oder -deletionen in eine Nukleotidsequenz aus Anhang A erzeugt werden, so daß eine oder mehrere Aminosäuresubstitutionen, -additionen oder -deletionen in das codierte Protein eingebracht werden. Die Mutationen können in eine der Sequenzen aus Anhang A durch Standard-Techniken, wie stellengerichtete Mutagenese und PCR-vermittelte Mutagenese, eingebracht werden. Vorzugsweise werden konservative Aminosäuresubstitutionen an einem oder mehreren der vorhergesagten nicht-essentiellen Aminosäurereste eingeführt. Bei einer "konservativen Aminosäuresubstitution" wird der Aminosäurerest durch einen Aminosäurerest mit einer ähnlichen Seitenkette ausgetauscht. Im Fachgebiet sind Familien von Aminosäureresten mit ähnlichen Seitenketten definiert worden. Diese Familien umfassen Aminosäuren mit basischen Seitenketten (z.B. Lysin, Arginin, Histidin), sauren Seitenketten (z.B. Asparaginsäure, Glutaminsäure), ungeladenen polaren Seitenketten (z.B. Glycin, Asparagin, Glutamin, Serin, Threonin, Tyrosin, Cystein), nicht-polaren Seitenketten, (bspw. Alanin, Valin, Leucin, Isoleucin, Prolin, Phenylalanin, Methionin, Tryptophan), betaverzweigten Seitenketten (z.B. Threonin, Valin, Isoleucin) und aromatischen Seitenketten (z.B. Tyrosin, Phenylalanin, Tryptophan, Histidin). Ein vorhergesagter nicht-essentieller Aminosäurerest in einem SMP-Protein wird somit vorzugsweise durch einen anderen Aminosäurerest der gleichen Seitenkettenfamilie ausgetauscht. In einer weiteren Ausführungsform können die Mutationen alternativ zufallsgemäß über die gesamte oder einen Teil der SMP-codierenden Sequenz eingebracht werden, bspw. durch Sättigungsmutagenese, und die resultierenden Mutanten können auf eine hier beschriebene SMP-Aktivität untersucht werden, um Mutanten zu identifizieren, die eine SMP-Aktivität beibehalten. Nach der Mutagenese von einer der Sequenzen aus Anhang A kann das codierte Protein rekombinant exprimiert werden, und die Aktivität des Proteins kann bspw. mit den hier beschriebenen Tests (siehe Beispiel 8 des Beispielteils) bestimmt werden.

### B. Rekombinante Expressionsvektoren und Wirtszellen

Ein weiterer Aspekt der Erfindung betrifft Vektoren, vorzugsweise Expressionsvektoren, die eine Nukleinsäure enthalten, die ein SMP-Protein (oder einen Abschnitt davon) codieren. Wie hier verwendet, betrifft der Begriff "Vektor" ein Nukleinsäuremolekül, das eine andere Nukleinsäure transportieren kann, an welche es gebunden ist. Ein Vektortyp ist ein "Plasmid", was für eine zirkuläre doppelsträngige DNA-Schleife steht, in die zusätzliche DNA-Segmente ligiert werden können. Ein weiterer Vektortyp ist ein viraler Vektor, wobei zusätzliche DNA-Segmente in das virale Genom ligiert werden können. Bestimmte Vektoren können in einer Wirtszelle, in die sie eingebracht worden sind, autonom replizieren (bspw. Bakterienvektoren, mit bakteriellem Replikationsursprung und episomale Säugetiervektoren). Andere Vektoren (z.B. nicht-episomale Säugetiervektoren) werden in das Genom einer Wirtszelle beim Einbringen in die Wirtszelle integriert und dadurch zusammen mit dem Wirtsgenom repliziert. Zudem können bestimmte Vektoren die Expression von Genen, mit denen sie funktionsfähig verbunden sind, steuern. Diese Vektoren werden hier als "Expressionsvektoren" bezeichnet. Gewöhnlich haben die Expressionsvektoren, die bei DNA-Rekombinationstechniken verwendet werden können, die Form von Plasmiden. In der vorliegenden Beschreibung können "Plasmid" und "Vektor" austauschbar verwendet werden, da das Plasmid die am häufigsten verwendete Vektorform ist. Die Erfindung soll jedoch diese anderen Expressionsvektorformen, wie virale Vektoren (bspw. replikationsdefiziente Retroviren, Adenoviren und adenoverwandte Viren), die ähnliche Funktionen ausüben, umfassen.

Die erfindungsgemäßen rekombinanten Expressionsvektoren umfassen eine erfindungsgemäße Nukleinsäure in einer Form, die sich zur Expression der Nukleinsäure in einer Wirtszelle eignet, d.h. daß die rekombinanten Expressionsvektoren eine oder mehrere regulatorische Sequenzen, ausgewählt auf der Basis der zur Expression zu verwendenden Wirtszellen, umfassen, die mit der zu exprimierenden Nukleinsäuresequenz funktionsfähig verbunden sind. In einem rekombinanten Expressionsvektor bedeutet "funktionsfähig verbunden", daß die Nukleotidsequenz von Interesse derart an die regulatorische(n) Sequenz(en) gebunden ist, daß die Expression der Nukleotidsequenz möglich ist (bspw. in einem in-vitro-Transkriptions-/Translationssystem oder in einer Wirtszelle, wenn der Vektor in die Wirtszelle eingebracht ist). Der Begriff "regulatorische Sequenz" soll Promotoren, Enhancer und andere Expressionskontrollelemente (bspw. Polyadenylierungssignale) umfassen. Diese regulatorischen Sequenzen sind bspw beschrieben in Goeddel: Gene Expression Technology: Methods in Enzymology 185, Academic Press, San Diego, CA (1990). Regulatorische Sequenzen umfassen solche, die die konstitutive Expression einer Nukleotidsequenz in vielen Wirtszelltypen steuern, und solche, die die Expression der Nukleotidsequenz nur in bestimmten Wirtszellen steuern. Der Fachmann ist sich dessen bewußt, daß die Gestaltung eines Expressionsvektors von Faktoren abhängen kann, wie der Wahl der zu transformierenden Wirtszelle, dem gewünschten Ausmaß der Proteinexpression usw. Die erfindungsgemäßen Expressionsvektoren können in die Wirtszellen eingebracht werden, so daß dadurch Proteine oder Peptide, einschließlich der Fusionsproteine oder -peptide, die von den Nukleinsäuren, wie hier beschrieben, codiert werden, hergestellt werden (bspw. SMP-Proteine, mutierte Formen von SMP-Proteinen, Fusionsproteine, usw.).

Die erfindungsgemäßen rekombinanten Expressionsvektoren können zur Expression von SMP-Proteinen in prokaryotischen oder eukaryotischen Zellen ausgestaltet sein. Bspw. können SMP-Gene in bakteriellen Zellen, wie *C*. *glutamicum,* Insektenzellen (mit Baculovirus-Expressionsvektoren), Hefe- und anderen Pilzzellen (siehe Romanos, M.A. et al. (1992) "Foreign gene expression in yeast: a review", Yeast 8: 423-488; van den Hondel, C.A.M.J.J. et al. (1991) "Heterologous gene expression in filamentous fungi" in: More Gene Manipulations in Fungi, J.W. Bennet & L.L. Lasure, Hrsg., S. 396-428: Academic Press: San Diego; und van den Hondel, C.A.M.J.J. & Punt, P.J. (1991) "Gene transfer systems and vector development for filamentous fungi. in: Applied Molecular Genetics of Fungi, Peberdy, J.F. et al., Hrsg, S. 1-28, Cambridge University Press: Cambridge), Algenzellen und Zellen vielzelliger Pflanzen (siehe Schmidt, R. und Willmitzer, L. (1988) "High efficiency Agrobacterium tumefaciens-mediated transformation of Arabidopsis thaliana leaf and cotyledon explants" Plant Cell Rep.: 583-586) oder Säugetierzellen exprimiert werden. Geeignete Wirtszellen werden weiter erörtert in Goeddel, Gene Expression Technology: Methods in Enzymology 185, Academic Press, San Diego, CA (1990). Der rekombinante Expressionsvektor kann alternativ, bspw. mit regulatorischen Sequenzen des T7-Promotors und T7-Polymerase, in vitro transkribiert und translatiert werden.

Die Expression von Proteinen in Prokaryoten erfolgt meist mit Vektoren, die konstitutive oder induzierbare Promotoren enthalten, die die Expression von Fusions- oder Nicht-Fusionsproteinen steuern. Fusionsvektoren steuern eine Reihe von Aminosäuren zu einem darin codierten Protein bei, gewöhnlich am Aminoterminus des rekombinanten Proteins, aber auch am C-Terminus oder fusioniert innerhalb geeigneter Bereiche der Proteine. Diese Fusionsvektoren haben gewöhnlich drei Aufgaben: 1) die Verstärkung der Expression von rekombinantem Protein; 2) die Erhöhung der Löslichkeit des rekombinanten Proteins; und 3) die Unterstützung der Reinigung des rekombinanten Proteins durch Wirkung als Ligand bei der Affinitätsreinigung. Bei Fusions-Expressionsvektoren wird oft eine proteolytische Spaltstelle an der Verbindungsstelle der Fusionseinheit und des rekombinanten Proteins eingebracht, so daß die Trennung des rekombinanten Proteins von der Fusionseinheit nach der Reinigung des Fusionsproteins möglich ist. Diese Enzyme und ihre entsprechenden Erkennungssequenzen umfassen Faktor Xa, Thrombin und Enterokinase.

Übliche Fusionsexpressionsvektoren umfassen pGEX (Pharmacia Biotech Inc; Smith, D.B. und Johnson, K.S. (1988) Gene 67:31-40), pMAL (New England Biolabs, Beverly, MA) und pRIT 5 (Pharmacia, Piscataway, NJ), bei denen Glutathion-S-Transferase (GST), Maltose E-bindendes Protein bzw. Protein A an das rekombinante Zielprotein fusioniert wird. Bei einer Ausführungsform ist die codierende Sequenz des SMP-Proteins in einen pGEX-Expressionsvektor kloniert, so daß ein Vektor erzeugt wird, der ein Fusionsprotein codiert, umfassend vom N-Terminus zum C-Terminus: GST - Thrombin-Spaltstelle - X-Protein. Das Fusionsprotein kann durch Affinitätschromatographie mittels Glutathion-Agarose-Harz gereinigt werden. Das rekombinante SMP-Protein, das nicht mit GST fusioniert ist, kann durch Spaltung des Fusionsproteins mit Thrombin gewonnen werden.

Beispiele geeigneter induzierbarer Nicht-Fusions-E.-coli-Expressionsvektoren umfassen pTrc (Amann et al., (1988) Gene 69:301-315) und pET 11d (Studier et al. Gene Expression Technology: Methods in Enzymology 185, Academic Press, San Diego, Kalifornien (1990) 60-89). Die Zielgenexpression aus dem pTrc-Vektor beruht auf der Transkription durch Wirts-RNA-Polymerase von einem Hybrid-trp-lac-Fusionspromotor. Die Zielgenexpression aus dem pET 11d-Vektor beruht auf der Transkription von einem T7-gn10-lac-Fusions-Promotor, die von einer coexprimierten viralen RNA-Polymerase (T7 gnl) vermittelt wird. Diese virale Polymerase wird von den Wirtsstämmen BL 21 (DE3) oder HMS174 (DE3) von einem residenten λ-Prophagen geliefert, der ein T7 gnl-Gen unter der Transkriptionskontrolle des lacUV 5-Promotors birgt.

Eine Strategie zur Maximierung der Expression des rekombinanten Proteins ist die Expression des Proteins in einem Wirtsbakterium, dessen Fähigkeit zur proteolytischen Spaltung des rekombinanten Proteins gestört ist (Gottesman, S. Gene Expression Technology: Methods in Enzymology 185, Academic Press, San Diego, Kalifornien (1990) 119-128). Eine weitere Strategie ist die Veränderung der Nukleinsäuresequenz der in einen Expressionsvektor zu inserierenden Nukleinsäure, so daß die einzelnen Codons für jede Aminosäure diejenigen sind, die vorzugsweise in einem zur Expression ausgewählten Bakterium, wie *C. glutamicum,* verwendet werden (Wada et al. (1992) Nucleic Acids Res. 20:2111-2118). Diese Veränderung der erfindungsgemäßen Nukleinsäuresequenzen kann durch Standard-DNA-Synthesetechniken erfolgen.

Bei einer weiteren Ausführungsform ist der SMP-Protein-Expressionsvektor ein Hefe-Expressionsvektor. Beispiele für Vektoren zur Expression in der Hefe *S*. *cerevisiae* umfassen pYepSec1 (Baldari et al., (1987) Embo J. 6:229-234), pMFa (Kurjan und Herskowitz (1982) Cell 30:933-943), pJRY88 (Schultz et al. (1987) Gene 54:113-123) sowie pYES2 (Invitrogen Corporation, San Diego, CA). Vektoren und Verfahren zur Konstruktion von Vektoren, die sich zur Verwendung in anderen Pilzen, wie filamentösen Pilzen, eignen, umfassen diejenigen, die eingehend beschrieben sind in: van den Hondel, C.A.M.J.J. & Punt, P.J. (1991) "Gene transfer systems and vector development for filamentous fungi, in: Applied Molecular Genetics of Fungi, J.F. Peberdy et al., Hrsg., S. 1-28, Cambridge University Press: Cambridge.

Alternativ können die erfindungsgemäßen SMP-Proteine in Insektenzellen unter Verwendung von Baculovirus-Expressionsvektoren exprimiert werden. Baculovirus-Vektoren, die zur Expression von Proteinen in gezüchteten Insektenzellen (bspw. Sf9-Zellen) verfügbar sind, umfassen die pAc-Reihe (Smith et al., (1983) Mol. Cell Biol., 3:2156-2165) und die pVL-Reihe (Lucklow und Summers (1989) Virology 170:31-39).

In einer weiteren Ausführungsform können die erfindungsgemäßen SMP-Proteine in Zellen einzelliger Pflanzen (wie Algen) oder in Pflanzenzellen höherer Pflanzen (bspw. Spermatophyten, wie Feldfrüchte) exprimiert werden. Beispiele für Pflanzen-Expressionsvektoren umfassen solche, die eingehend beschrieben sind in: Bekker, D., Kemper, E., Schell, J. und Masterson, R. (1992) "New plant binary vectors with selectable markers located proximal to the left border", Plant Mol. Biol. 20:1195-1197; und Bevan, M.W. (1984)."Binary Agrobacterium vectors for plant transformation", Nucl. Acids Res. 12:8711-8721.

In einer weiteren Ausführungsform wird eine erfindungsgemäße Nukleinsäure in Säugetierzellen mit einem Säugetier-Expressionsvektor exprimiert. Beispiele für Säugetier-Expressionsvektoren umfassen pCDM8 (Seed, B. (1987) Nature 329:840) und pMT2PC (Kaufman et al. (1987) EMBO J. 6:187-195). Bei der Verwendung in Säugetierzellen werden die Kontrollfunktionen des Expressionsvektors oft von viralen regulatorischen Elementen bereitgestellt. Gemeinhin verwendete Promotoren stammen bspw. aus Polyoma, Adenovirus 2, Cytomegalievirus und Simian Virus 40. Weitere geeignete Expressionssysteme für prokaryotische und eukaryotische Zellen siehe in Kapitel 16 und 17 von Sambrook, J., Fritsch, E.F. und Maniatis, T., Molecular cloning: A Laboratory Manual, 2. Auflage, Cold Spring Harbor Laboratory, Cold Spring Harbor Laboratory Press, Cold Spring Harbor, NY, 1989.

Bei einer weiteren Ausführungsform kann der rekombinante Säugetier-Expressionsvektor die Expression der Nukleinsäure vorzugsweise in einem bestimmten Zelltyp bewirken (bspw. werden gewebespezifische regulatorische Elemente zur Expression der Nukleinsäure verwendet). Gewebespezifische regulatorische Elemente sind im Fachgebiet bekannt. Nicht-einschränkende Beispiele für geeignete gewebespezifische Promotoren umfassen den Albuminpromotor (leberspezifisch; Pinkert et al. (1987) Genes Dev. 1:268-277), lymphoid-spezifische Promotoren (Calame und Eaton (1988) Adv. Immunol. 43:235-275), insbesondere Promotoren von T-Zellrezeptoren (Winoto und Baltimore (1989) EMBO J. 8:729-733) und Immunglobulinen (Banerji et al. (1983) Cell 33:729-740; Queen und Baltimore (1983) Cell 33:741-748), neuronenspezifische Promotoren (bspw. der Neurofilament-Promotor; Byrne und Ruddle (1989) PNAS 86:5473-5477), pankreasspezifische Promotoren (Edlund et al., (1985) Science 230:912-916) und milchdrüsenspezifische Promotoren (bspw. Milchserum-Promotor; US-Patent Nr. 4 873 316 und europäische Patentanmeldungsveröffentlichung Nr. 264 166). Entwicklungsregulierte Promotoren sind ebenfalls umfaßt, bspw. die Maus-hox-Promotoren (Kessel und Gruss (1990) Science 249:374-379) und der α-Fetoprotein-Promotor (Campes und Tilghman (1989) Genes Dev. 3:537-546).

Beschrieben werden rekombinanten Expressionsvektoren umfassend ein erfindungsgemäßes DNA Molekül, das in Antisense-Richtung in den Expressionsvektor kloniert ist. D.h. daß das DNA-Molekül derart mit einer regulatorischen Sequenz funktionsfähig verbunden ist, daß die Expression (durch Transkription des DNA-Moleküls) eines RNA-Moleküls, das zur SMP-mRNA antisense ist, möglich wird. Es können regulatorische Sequenzen ausgewählt werden, die funktionsfähig an eine in Antisense-Richtung klonierte Nukleinsäure gebunden sind und die kontinuierliche Expression des Antisense-RNA-Moleküls in einer Vielzahl von Zelltypen steuern, bspw. können virale Promotoren und/oder Enhancer oder regulatorische Sequenzen ausgewählt werden, die die konstitutive, gewebespezifische oder zelltypspezifische Expression von Antisense-RNA steuern. Der Antisense-Expressionsvektor kann in Form eines rekombinanten Plasmids, Phagemids oder attenuierten Virus vorliegen, in dem Antisense-Nukleinsäuren unter der Kontrolle eines hochwirksamen regulatorischen Bereichs produziert werden, dessen Aktivität durch den Zelltyp bestimmt wird, in den der Vektor eingebracht wird. Für eine Diskussion der Regulation der Genexpression mittels Antisense-Genen siehe Weintraub, H. et al., Antisense-RNA as a molecular tool for genetic analysis, Reviews - Trends in Genetics, Bd. 1(1) 1986.

Ein weiterer Aspekt der Erfindung betrifft die Wirtszellen, in die ein erfindungsgemäßer rekombinanter Expressionsvektor eingebracht worden ist. Die Begriffe "Wirtszelle" und "rekombinante Wirtszelle" werden hier untereinander austauschbar verwendet. Es ist selbstverständlich, daß diese Begriffe nicht nur eine bestimmte Zielzelle, sondern auch die Nachkommen oder potentiellen Nachkommen dieser Zelle betreffen. Da in aufeinanderfolgenden Generationen aufgrund von Mutation oder Umwelteinflüssen bestimmte Modifikationen auftreten können, sind diese Nachkommen nicht unbedingt mit der Parentalzelle identisch, sind jedoch im Umfang des Begriffs, wie er hier verwendet wird, noch umfaßt.

Eine Wirtszelle kann eine prokaryotische oder eukaryotische Zelle sein. Bspw. kann ein SMP-Protein in Bakterienzellen, wie *C. glutamicum,* Insektenzellen, Hefe- oder Säugetierzellen (wie Ovarzellen des chinesischen Hamsters (CHO) oder COS-Zellen) exprimiert werden. Andere geeignete Wirtszellen sind dem Fachmann geläufig. Mikroorganismen, die mit *Corynebacterium glutamicum* verwandt sind und eignen sich als Wirtszellen für die erfindungsgemäßen Nukleinsäure- und Proteinmoleküle.

Durch herkömmliche Transformations- oder Transfektionsverfahren läßt sich Vektor-DNA in prokaryotische oder eukaryotische Zellen einbringen. Die Begriffe "Transformation" und "Transfektion", "Konjugation" und "Transduktion", wie sie hier verwendet werden, sollen eine Vielzahl von im Stand der Technik bekannten Verfahren zum Einbringen fremder Nukleinsäure (bspw. DNA) in eine Wirtszelle umfassen, einschließlich Calciumphosphat- oder Calciumchlorid-Copräzipitation, DEAE-Dextran-vermittelter Transfektion, Lipofektion, natürlicher Kompetenz, chemisch vermittelter Übertragung oder Elektroporation. Geeignete Verfahren zur Transformation oder Transfektion von Wirtszellen lassen sich nachlesen in Sambrook et al. (Molecular Cloning: A Laboratory Manual. 2. Aufl., Cold Spring Harbor Laboratory, Cold Spring Harbor Laboratory Press, Cold Spring Harbor, NY, 1989) und anderen Labor-Handbüchern.

Es ist bekannt, daß für die stabile Transfektion von Säugetierzellen je nach dem verwendeten Expressionsvektor und der verwendeten Transfektionstechnik nur ein kleiner Teil der Zellen die fremde DNA in ihr Genom integrieren kann. Zur Identifizierung und Selektion dieser Integranten wird gewöhnlich ein Gen, das einen selektierbaren Marker (z.B. Resistenz gegen Antibiotika) codiert, zusammen mit dem Gen von Interesse in die Wirtszellen eingebracht. Bevorzugte selektierbare Marker umfassen solche, die die Resistenz gegen Medikamente, wie G418, Hygromycin und Methotrexat, verleihen. Eine Nukleinsäure, die einen selektierbaren Marker codiert, kann in eine Wirtszelle auf dem gleichen Vektor eingebracht werden, wie derjenige, der ein SMP-Protein codiert, oder kann auf einem gesonderten Vektor eingebracht werden. Zellen, die mit der eingebrachten Nukleinsäure stabil transfiziert worden sind, können bspw. durch Medikamentenselektion identifiziert werden (z.B. überleben Zellen, die den selektierbaren Marker integriert haben, wohingegen die anderen Zellen sterben).

Zur Erzeugung eines homolog rekombinierten Mikroorganismus wird ein Vektor hergestellt, der zumindest einen Abschnitt eines SMP-Gens enthält, in den eine Deletion, Addition oder Substitution eingebracht worden ist, um das SMP-Gen zu verändern, bspw. funktionell zu disruptieren. Dieses SMP-Gen ist vorzugsweise ein Co*rynebacterium glutamicum*-SMP-Gen, jedoch kann ein Homologon von einem verwandten Bakterium oder sogar aus einer Säugetier-, Hefe- oder Insektenquelle verwendet werden. Bei einer bevorzugten Ausführungsform ist der Vektor derart ausgestaltet, daß das endogene SMP-Gen bei homologer Rekombination funktionell disrumpiert ist (d.h. nicht länger ein funktionelles Protein codiert; auch als "Knockout"-Vektor bezeichnet). Der Vektor kann alternativ derart ausgestaltet sein, daß das endogene SMP-Gen bei homologer Rekombination mutiert oder anderweitig verändert ist, jedoch noch das funktionelle Protein codiert (z.B. kann der stromaufwärts gelegene regulatorische Bereich derart verändert sein, daß dadurch die Expression des endogenen SMP-Proteins verändert wird.). Der veränderte Abschnitt des SMP-Gens ist im homologen Rekombinationsvektor an seinem 5'- und 3'-Ende von zusätzlicher Nukleinsäure des SMP-Gens flankiert, die eine homologe Rekombination zwischen dem exogenen SMP-Gen, das von dem Vektor getragen wird, und einem endogenen SMP-Gen in einem Mikroorganismus ermöglicht. Die zusätzliche flankierende SMP-Nukleinsäure ist für eine erfolgreiche homologe Rekombination mit dem endogenen Gen hinreichend lang. Gewöhnlich enthält der Vektor mehrere Kilobasen flankierende DNA (sowohl am 5'- als auch am 3'-Ende) (siehe z.B. Thomas, K.R. und Capecchi, M.R. (1987) Cell 51:503 für eine Beschreibung von homologen Rekombinationsvektoren). Der Vektor wird in einen Mikroorganismus (z.B. durch Elektroporation) eingebracht, und Zellen, in denen das eingebrachte SMP-Gen mit dem endogenen SMP-Gen homolog rekombiniert ist, werden unter Verwendung im Fachgebiet bekannter Verfahren selektiert.

Bei einer anderen Ausführungsform können rekombinante Mikroorganismen produziert werden, die ausgewählte Systeme enthalten, die eine regulierte Expression des eingebrachten Gens ermöglichen. Der Einschluß eines SMP-Gens in einen Vektor, wodurch es unter die Kontrolle des Lac-Operons gebracht wird, ermöglicht z.B. die Expression des SMP-Gens nur in Gegenwart von IPTG. Diese regulatorischen Systeme sind im Fachgebiet bekannt.

Eine erfindungsgemäße Wirtszelle, wie eine prokaryotische oder eukaryotische Wirtszelle in Kultur, kann zur Produktion (d.h. Expression) eines SMP-Proteins verwendet werden. Die Erfindung stellt zudem Verfahren zur Produktion von SMP-Proteinen unter Verwendung der erfindungsgemäßen Wirtszellen bereit. Bei einer Ausführungsform umfaßt das Verfahren die Anzucht der erfindungsgemäßen Wirtszelle (in die ein rekombinanter Expressionsvektor, der ein SMP-Protein codiert, eingebracht worden ist, oder in deren Genom ein Gen eingebracht worden ist, das ein Wildtyp- oder verändertes SMP-Protein codiert) in einem geeigneten Medium, bis das SMP-Protein produziert worden ist. Das Verfahren umfaßt in einer weiteren Ausführungsform das Isolieren der SMP-Proteine aus dem Medium oder der Wirtszelle.

### C. Erfindungsgemäße Verwendungen und Verfahren

Die hier beschriebenen Nukleinsäuremoleküle, Proteine, Proteinhomologa, Fusionsproteine, Primer, Vektoren und Wirtszellen können in einem oder mehreren nachstehenden Verfahren verwendet werden:

Identifikation von *C. glutamicum* und verwandten Organismen, Kartierung von Genomen von Organismen, die mit *C. glutamicum* verwandt sind, Identifikation und Lokalisation von *C. glutamicum*-Sequenzen von Interesse, Evolutionsstudien, Bestimmung von SMP-Proteinbereichen, die für die Funktion notwendig sind, Modulation der Aktivität eines SMP-Proteins; Modulation des Stoffwechsels eines oder mehrerer Zucker; Modulation der Produktion energiereicher Moleküle in einer Zelle (d.h. von ATP, NADPH) und Modulation der zellulären Produktion einer gewünschten Verbindung, wie einer Feinchemikalie. Die erfindungsgemäßen SMP-Nukleinsäuremoleküle haben eine Vielzahl von Verwendungen. Sie können zunächst zur Identifikation eines Organismus als *Corynebacterium glutamicum* oder naher Verwandter davon verwendet werden. Sie können zudem zur Identifikation des Vorliegens von *C*. *glutamicum* oder eines Verwandten davon in einer Mischpopulation von Mikroorganismen verwendet werden. Die Erfindung stellt die Nukleinsäuresequenzen einer Reihe von *C*. *glutamicum*-Genen bereit. Durch Sondieren der extrahierten genomischen DNA einer Kultur einer einheitlichen oder gemischten Population von Mikroorganismen unter stringenten Bedingungen mit einer Sonde, die einen Bereich eines *C. glutamicum*-Gens überspannt, das für diesen Organismus einzigartig ist, kann man bestimmen, ob dieser Organismus zugegen ist. *Corynebacterium glutamicum* selbst ist zwar nicht pathogen, jedoch ist es mit pathogenen Arten, wie *Corynebacterium diptheriae*, verwandt. Der Nachweis eines solchen Organismus ist von signifikanter klinischer Bedeutung.

Die erfindungsgemäßen Nukleinsäure- und Proteinmoleküle können ferner als Marker für spezifische Bereiche des Genoms dienen. Dies ist nicht nur beim Kartieren des Genoms, sondern auch für funktionelle Studien von *C. glutamicum-*Proteinen nützlich. Zur Identifikation des Genombereichs, an den ein bestimmtes *C. glutamicum*-DNA-bindendes Protein bindet, kann das *C*. *glutamicum*-Genom gespalten und die Fragmente mit dem DNA-bindenden Protein inkubiert werden. Diejenigen, die das Protein binden, können zusätzlich mit den erfindungsgemäßen Nukleinsäuremolekülen, vorzugsweise mit leicht nachweisbaren Markierungen, sondiert werden; die Bindung eines solchen Nukleinsäuremoleküls an das Genomfragment ermöglicht die Lokalisation des Fragmentes auf der genomischen Karte von *C*. *glutamicum,* und wenn dies mehrmals mit unterschiedlichen Enzymen durchgeführt wird, erleichtert es eine rasche Bestimmung der Nukleinsäuresequenz, an die das Protein bindet. Die erfindungsgemäßen Nukleinsäuremoleküle können zudem hinreichend homolog zu den Sequenzen verwandter Arten sein, so daß diese Nukleinsäuremoleküle als Marker für die Konstruktion einer genomischen Karte in verwandten Bakterien, wie *Brevibacterium lactofer*mentum, dienen können.

Die erfindungsgemäßen SMP-Nukleinsäuremoleküle eignen sich ebenfalls für Evolutions- und Proteinstruktur-Untersuchungen. Die Stoffwechsel- und Energiefreisetzungsprozesse, an denen die erfindungsgemäßen Moleküle beteiligt sind, werden von einer Vielzahl von prokaryotischen und eukaryotischen Zellen ausgenutzt; durch Vergleich der Sequenzen der erfindungsgemäßen Nukleinsäuremoleküle mit solchen, die ähnliche Enzyme aus anderen Organismen codieren, kann der Evolutions-Verwandschaftsgrad der Organismen bestimmt werden. Entsprechend ermöglicht ein solcher Vergleich die Bestimmung, welche Sequenzbereiche konserviert sind und welche nicht, was bei der Bestimmung solcher Bereiche des Proteins hilfreich sein kann, die für die Enzymfunktion essentiell sind. Dieser Typ der Bestimmung ist für Proteintechnologie-Untersuchungen wertvoll und kann einen Hinweis darauf geben, wieviel Mutagenese das Protein tolerieren kann ohne die Funktion zu verlieren.

Die Manipulation der erfindungsgemäßen SMP-Nukleinsäuremoleküle kann die Produktion von SMP-Proteinen mit funktionellen Unterschieden zu den Wildtyp-SMP-Proteinen bewirken. Diese Proteine können hinsichtlich ihrer Effizienz oder Aktivität verbessert werden, können in größerer Anzahl als gewöhnlich in der Zelle zugegen sein oder können hinsichtlich ihrer Effizienz oder Aktivität geschwächt sein.

Es gibt eine Reihe von Mechanismen, durch die die Änderung eines erfindungsgemäßen SMP-Proteins die Ausbeute, Produktion und/oder Effizienz der Produktion einer Feinchemikalie aus einem *C*. *glutamicum*-Stamm, der ein solches verändertes Protein enthält, direkt beeinflussen kann. Der Abbau von energiereichen Kohlenstoffmolekülen, wie Zuckern, und die Umwandlung von Verbindungen, wie NADH und FADH₂ über die oxidative Phosphorylierung in nützlichere Verbindungen führt zu einer Reihe von Verbindungen, die selbst wünschenswerte Feinchemikalien sein können, wie Pyruvat, ATP, NADH und eine Reihe von Zucker-Zwischenverbindungen. Ferner werden die Energiemoleküle (wie ATP) und die Reduktionsäquivalente (wie NADH oder NADPH), die durch diese Stoffwechselwege produziert werden, in der Zelle zum Antreiben von Reaktionen verwendet, die ansonsten energetisch ungünstig wären. Zu solchen ungünstigen Reaktionen gehören viele Biosynthesewege von Feinchemikalien. Durch die Verbesserung der Fähigkeit der Zelle, einen bestimmten Zucker zu nutzen (z.B. durch Manipulation der Gene, die am Abbau und an der Umwandlung des Zuckers in Energie für die Zelle beteiligt sind), läßt sich die Menge an Energie, die verfügbar ist, damit ungünstige und trotzdem gewünschte Stoffwechselreaktionen (z.B. die Biosynthese einer gewünschten Feinchemikalie) auftreten, vergrößern.

Ferner ermöglicht die Modulierung eines oder mehrerer Wege, die an der Zuckernutzung beteiligt sind, die Optimierung der Umwandlung der Energie, die im Zuckermolekül enthalten ist, zur Produktion einer oder mehrerer Feinchemikalien. Beispielsweise ist durch Reduktion der Aktivität von Enzymen, die z.B. an der Guconeogenese beteiligt sind, mehr ATP zum Antreiben gewünschter biochemischer Reaktionen (wie der Feinchemikalien-Biosynthese) in der Zelle verfügbar. Auch die Gesamtproduktion von Energiemolekülen aus Zuckern kann moduliert werden, damit gewährleistet ist, daß die Zelle ihre Energieproduktion aus jedem Zuckermolekül maximiert. Eine ineffiziente Zuckernutzung kann zu übermäßiger CO₂-Produktion und überschüssiger Energie führen, die zu Leerlauf-Stoffwechselzyklen führen können. Durch Verbesserung des Stoffwechsels von Zuckermolekülen sollte die Zelle effizienter arbeiten können und weniger Kohlenstoffmoleküle verbrauchen. Dies sollte zu einem verbesserten Feinchemikalienprodukt:Zuckermolekül-Verhältnis (verbesserter Kohlenstoffausbeute) führen und ermöglicht eine Verringerung der Menge an Zuckern, die in einer Fermenterkultur im Großmaßstab derart veränderter *C*. *glutamicum* zum Medium gegeben werden müssen.

Die Mutagenese von einem oder mehreren erfindungsgemäßen SMP-Proteinen kann auch zu SMP-Proteinen mit geänderten Aktivitäten führen, die indirekt die Produktion einer oder mehrerer gewünschten Feinchemikalien aus *C. glutamicum* beeinflussen. Beispielsweise kann man durch Erhöhen der Effizienz der Nutzung eines oder mehrerer Zucker (so daß die Umwandlung des Zuckers in nutzbare Energiemoleküle verbessert ist) oder durch Erhöhen der Effizienz der Umwandlung von Reduktionsäquivalenten in nutzbare Energiemoleküle (z.B. durch Verbesserung der Effizienz der oxidativen Phosphorylierung oder der Aktivität der ATP-Synthase) die Menge dieser energiereichen Verbindungen, die für die Zelle zum Antrieb normalerweise ungünstiger Stoffwechselprozesse verfügbar ist, steigern. Zu diesen Prozessen gehört der Aufbau der Zellwände, die Transkription, Translation und die Biosynthese von Verbindungen, die für das Wachstum und die Teilung von Zellen nötig sind (z.B. Nukleotide, Aminosäuren, Vitamine, Lipide usw.) (Lengeler et al. (1999) Biology of Prokaryotes, Thieme Verlag: Stuttgart, S. 88-109; 913-918; 875-899). Durch Verbessern von Wachstum und Vermehrung dieser veränderten Zellen ist es möglich, die Lebensfähigkeit der Zellen in Kulturen im Großmaßstab zu steigern und auch ihre Teilungsrate zu verbessern, so daß eine vergleichsweise größere Anzahl Zellen in der Fermenterkultur überleben kann. Die Ausbeute, Produktion oder Effizienz der Produktion kann zumindest aufgrund der Anwesenheit einer größeren Anzahl lebensfähiger Zellen, die jeweils die gewünschte Feinchemikalie produzieren, erhöht werden.

Ferner werden viele der Abbauprodukte, die während des Zuckermetabolismus produziert werden, selbst durch die Zelle als Vorstufen oder Zwischenprodukte zur Bildung einer Reihe anderer nützlicher Verbindungen verwendet, von denen einige selbst Feinchemikalien sind. Beispielsweise wir Pyruvat in die Aminosäure Alanin umgewandelt, und Ribose-5-phosphat ist integraler Bestandteil von zum Beispiel Nukelotidmolekülen. Das Ausmaß und die Effizienz des Zuckermetabolismus hat dann eine grundlegende Auswirkung auf die Verfügbarkeit dieser Abbauprodukte in der Zelle. Durch Steigern der Fähigkeit der Zelle, Zucker zu verarbeiten, entweder über die Effizienz bestehender Wege (z.B. durch Veränderung von Enzymen, die an diesen Wegen beteiligt sind, so daß ihre Aktivität optimiert ist) oder durch Erhöhen der Verfügbarkeit der an diesen Wegen beteiligten Enzyme (z.B. durch Vergrößern der Anzahl der in der Zelle vorliegenden Enzyme) ist es möglich, die Verfügbarkeit dieser Abbauprodukte in der Zelle zu steigern, was wiederum die Produktion von vielen verschiedenen anderen wünschenswerten Verbindungen in der Zelle (z.B. Feinchemikalien) erhöhen sollte.

Diese vorstehend genannten Mutagenesestrategien für SMP-Proteine, die erhöhte Ausbeuten einer Feinchemikalie aus *C. glutamicum* bewirken sollen, sollen nicht einschränkend sein; Variationen dieser Mutagenesestrategien sind dem Fachmann leicht ersichtlich. Unter Verwendung dieser Strategien und einschließlich der hier offenbarten Mechanismen können die erfindungsgemäßen Nukleinsäure- und Proteinmoleküle verwendet werden, um *C. glutamicum-* oder verwandte Bakterienstämme, die mutierte SMP-Nukleinsäure- und Proteinmoleküle exprimieren, zu erzeugen, so daß die Ausbeute, Produktion und/oder Effizienz der Produktion einer gewünschten Verbindung verbessert wird. Die gewünschte Verbindung kann jedes von *C*. *glutamicum* hergestellte Produkt sein, einschließlich der Endprodukte von Biosynthesewegen und Zwischenprodukte natürlich vorkommender metabolischer Wege sowie Moleküle, die im Metabolismus von *C. glutamicum* nicht natürlich vorkommen, die jedoch von einem erfindungsgemäßen *C. glutamicum-*Stamm produziert werden.

Diese Erfindung wird durch die nachstehenden Beispiele weiter veranschaulicht, die nicht als einschränkend aufgefaßt werden sollen.

### Beispiele

### Beispiel 1: Präparation der gesamten genomischen DNA aus Corynebacterium glutamicum ATCC13032

Eine Kultur von *Corynebacterium glutamicum* (ATCC 13032) wurde über Nacht bei 30°C unter starkem Schütteln in BHI-Medium (Difco) gezüchtet. Die Zellen wurden durch Zentrifugation geerntet, der Überstand wurde verworfen, und die Zellen wurden in 5ml Puffer I (5% des Ursprungsvolumens der Kultur - sämtliche angegebenen Volumina sind für 100 ml Kulturvolumen berechnet) resuspendiert. Zusammensetzung von Puffer I: 140,34 g/l Saccharose, 2,46 g/l MgSO₄ · 7 H₂O, 10 ml/l KH₂PO₄-Lösung (100g/l, mit KOH auf pH-Wert 6,7 eingestellt), 50 ml/l M12-Konzentrat (10 g/l (NH₄)₂SO₄, 1 g/l NaCl, 2 g/l MgSO₄ · 7 H₂O, 0,2 g/l CaCl₂, 0,5 g/l Hefe-Extrakt (Difco), 10 ml/l Spurenelemente-Mischung (200 mg/l FeSO₄ · H₂O, 10 mg/l ZnSO₄ · 7 H₂O, 3 mg/l MnCl₂ · 4 H₂O, 30 mg/l H₃BO₃, 20 mg/l CoCl₂ · 6 H₂O, 1 mg/l NiCl₂ · 6 H₂O, 3 mg/l Na₂MoO₄ · 2 H₂O, 500 mg/l Komplexbildner (EDTA oder Citronensäure), 100 ml/l Vitamingemisch (0,2 ml/l Biotin, 0,2 mg/l Folsäure, 20 mg/l p-Aminobenzoesäure, 20 mg/l Riboflavin, 40 mg/l Ca-Panthothenat, 140 mg/l Nikotinsäure, 40 mg/l Pyridoxolhydrochlorid, 200 mg/l Myo-Inositol). Lysozym wurde in einer Endkonzentration von 2,5 mg/ml zur Suspension gegeben. Nach etwa 4 Std. Inkubation bei 37°C wurde die Zellwand abgebaut, und die erhaltenen Protoplasten wurden durch Zentrifugation geerntet. Das Pellet wurde einmal mit 5 ml Puffer I und einmal mit 5 ml TE-Puffer (10 mM Tris-HCl, 1 mM EDTA, pH-Wert 8) gewaschen. Das Pellet wurde in 4 ml TE-Puffer resuspendiert, und 0,5 ml SDS-Lösung (10%) und 0,5 ml NaCl-Lösung (5 M) wurden zugegeben. Nach Zugabe von Proteinase K in einer Endkonzentration von 200 µg/ml wurde die Suspension etwa 18 Std. bei 37°C inkubiert. Die DNA wurde durch Extraktion mit Phenol, Phenol-Chloroform-Isoamylalkohol und Chloroform-Isoamylalkohol mittels Standard-Verfahren gereinigt. Dann wurde die DNA durch Zugabe von 1/50 Volumen 3 M Natriumacetat und 2 Volumina Ethanol, anschließender Inkubation für 30 min bei -20°C und 30 min Zentrifugation bei 12000 U/min in einer Hochgeschwindigkeitszentrifuge mit einem SS34-Rotor (Sorvall) gefällt. Die DNA wurde in 1 ml TE-Puffer gelöst, der 20 µg/ml RNase A enthielt, und für mindestens 3 Std. bei 4°C gegen 1000 ml TE-Puffer dialysiert. Während dieser Zeit wurde der Puffer 3mal ausgetauscht. Zu Aliquots von 0,4 ml der dialysierten DNA-Lösung wurden 0,4 ml 2 M LiCl und 0,8 ml Ethanol zugegeben. Nach 30 min Inkubation bei -20°C wurde die DNA durch Zentrifugation gesammelt (13000 U/min, Biofuge Fresco, Heraeus, Hanau, Deutschland). Das DNA-Pellet wurde in TE-Puffer gelöst. Durch dieses Verfahren hergestellte DNA konnte für alle Zwecke verwendet werden, einschließlich Southern-Blotting oder zur Konstruktion genomischer Banken.

### Beispiel 2: Konstruktion genomischer Corynebacterium glutamicum (ATCC13032)-Banken in Escherichia coli

Ausgehend von DNA, die wie in Beispiel 1 beschrieben hergestellt wurde, wurden gemäß bekannter und gut eingeführter Verfahren (siehe bspw. Sambrook, J. et al. (1989) "Molecular Cloning: A Laboratory Manual". Cold Spring Harbor Laboratory Press oder Ausubel, F.M. et al. (1994) "Current Protocols in Molecular Biology", John Wiley & Sons) Cosmid- und Plasmid-Banken hergestellt.

Es ließ sich jedes Plasmid oder Cosmid einsetzen. Besondere Verwendung fanden die Plasmide pBR322 (Sutcliffe, J.G. (1979) Proc. Natl Acad. Sci. USA, 75:3737-3741); pACYC177 (Change & Cohen (1978) J. Bacteriol. 134:1141-1156); Plasmide der pBS-Reihe (pBSSK+, pBSSK- und andere; Stratagene, LaJolla, USA) oder Cosmide, wie SuperCos1 (Stratagene, LaJolla, USA) oder Lorist6 (Gibson, T.J. Rosenthal, A., und Waterson, R.H. (1987) Gene 53: 283-286.

### Beispiel 3: DNA-Sequenzierung und Computer-Funktionsanalyse

Genomische Banken, wie in Beispiel 2 beschrieben, wurden zur DNA-Sequenzierung gemäß Standard-Verfahren, insbesondere dem Kettenabbruchverfahren mit ABI377-Sequenziermaschinen (s. z.B. Fleischman, R.D. et al. (1995) "Whole-genome Random Sequencing and Assembly of Haemophilus Influenzae Rd., Science 269:496-512) verwendet. Die Sequenzierprimer mit den folgenden Nukleotidsequenzen wurden verwendet: 5'-GGAAACAGTATGACCATG-3' oder 5'-GTAAAACGACGGCCAGT-3'.

### Beispiel 4: In-vivo-Mutagenese

In vivo-Mutagenese von *Corynebacterium glutamicum* kann durchgeführt werden, indem eine Plasmid- (oder andere Vektor-) DNA durch *E. coli* oder andere Mikroorganismen (z.B. *Bacillus* spp. oder Hefen, wie *Saccharomyces cerevisiae*) geschleust wird, die die Integrität ihrer genetischen Information nicht aufrechterhalten können. Übliche Mutatorstämme weisen Mutationen in den Genen für das DNA-Reparatursystem auf (z.B., mutHLS, mutD, mutT, usw., zum Vergleich siehe Rupp, W.D. (1996) DNA repair mechanisms, in: Escherichia coli and Salmonella, S. 2277-2294, ASM: Washington). Diese Stämme sind dem Fachmann bekannt. Die Verwendung dieser Stämme ist bspw. in Greener, A. und Callahan, M. (1994) Strategies 7:32-34 veranschaulicht.

### Beispiel 5: DNA-Transfer zwischen Escherichia coli und Corynebacterium glutamicum

Mehrere *Corynebacterium-* und *Brevibacterium*-Arten enthalten endogene Plasmide (wie bspw. pHM1519 oder pBL1) die autonom replizieren (für einen Überblick siehe bspw. Martin, J.F. et al. (1987) Biotechnology 5:137-146). Shuttle-Vektoren für *Escherichia coli* und *Corynebacterium glutamicum* lassen sich leicht mittels Standard-Vektoren für *E. coli* konstruieren (Sambrook, J. et al., (1989), "Molecular Cloning: A Laboratory Manual", Cold Spring Harbor Laboratory Press oder Ausubel, F.M. et al. (1994) "Current Protocols in Molecular Biology", John Wiley & Sons), denen ein Replikationsursprung für und ein geeigneter Marker aus *Corynebacterium glutamicum* beigegeben wird. Solche Replikationsursprünge werden vorzugsweise von endogenen Plasmiden entnommen, die aus *Corynebacterium-* und *Brevibactertium*-Arten isoliert worden sind. Besondere Verwendung als Transformationsmarker für diese Arten sind Gene für Kanamycin-Resistenz (wie solche, die vom Tn5- oder Tn-903-Transposon stammen) oder für Chloramphenicol (Winnacker, E.L. (1987) "From Genes to Clones - Introduction to Gene Technology, VCH, Weinheim). Es gibt zahlreiche Beispiele in der Literatur für die Herstellung einer großen Vielzahl von Shuttle-Vektoren, die in *E*. *coli* und *C*. *glutamicum* replizieren und für verschiedene Zwecke verwendet werden können, einschließlich Gen-Überexpression (siehe bspw. Yoshihama, M. et al. (1985) J. Bacteriol. 162:591-597, Martin, J.F. et al., (1987) Biotechnology, 5:137-146 und Eikmanns, B.J. et al. (1992) Gene 102:93-98).

Mittels Standard-Verfahren ist es möglich, ein Gen von Interesse in einen der vorstehend beschriebenen Shuttle-Vektoren zu klonieren und solche Hybrid-Vektoren in *Corynebacterium glutamicum-*Stämme einzubringen. Die Transformation von *C*. *glutamicum* läßt sich durch Protoplastentransformation (Kastsumata, R. et al., (1984) J. Bacteriol. 159:306-311), Elektroporation (Liebl, E. et al., (1989) FEMS Microbiol. Letters, 53:399-303) und in Fällen, bei denen spezielle Vektoren verwendet werden, auch durch Konjugation erzielen (wie z.B. beschrieben in Schäfer, A., et (1990) J. Bacteriol. 172:1663-1666). Es ist ebenfalls möglich, die Shuttle-Vektoren für *C. glutamicum* auf *E. coli* zu übertragen, indem Plasmid-DNA aus *C*. *glutamicum* (mittels im Fachgebiet bekannter Standard-Verfahren) präpariert und in *E*. *coli* transformiert wird. Dieser Transformationsschritt kann mit Standard-Verfahren erfolgen, jedoch wird vorteilhafterweise ein Mcr-defizienter *E*. *coli*-Stamm verwendet, wie NM522 (Gough & Murray (1983) J. Mol. Biol. 166:1-19).

### Beispiel 6: Bestimmung der Expression des mutanten Proteins

Die Beobachtungen der Aktivität eines mutierten Proteins in einer transformierten Wirtszelle beruhen auf der Tatsache, daß das mutante Protein auf ähnliche Weise und in ähnlicher Menge exprimiert wird wie das Wildtyp-Protein. Ein geeignetes Verfahren zur Bestimmung der Transkriptionsmenge des mutanten Gens (ein Anzeichen für die mRNA-Menge, die für die Translation des Genprodukts verfügbar ist) ist die Durchführung eines Northern-Blots (s. bspw. Ausubel et al., (1988) Current Protocols in Molecular Biology, Wiley: New York), wobei ein Primer, der so ausgestaltet ist, daß er an das Gen von Interesse bindet, mit einer nachweisbaren (gewöhnlich radioaktiven oder chemilumineszierenden) Markierung versehen wird, so daß - wenn die Gesamt-RNA einer Kultur des Organismus extrahiert, auf einem Gel aufgetrennt, auf eine stabile Matrix übertragen und mit dieser Sonde inkubiert wird - die Bindung und die Quantität der Bindung der Sonde das Vorliegen und auch die Menge der mRNA für dieses Gen anzeigt. Diese Information ist ein Hinweis auf das Ausmaß der Transkription des mutanten Gens. Gesamt-Zell-RNA läßt sich durch verschiedene Verfahren aus *Corynebacterium glutamicum* isolieren, die im Fachgebiet bekannt sind, wie in Bormann, E.R. et al., (1992) Mol. Microbiol. 6:317-326 beschrieben.

Zur Bestimmung des Vorliegens oder der relativen Menge an Protein, das von dieser mRNA translatiert wird, können Standard-Techniken, wie Western-Blot, eingesetzt werden (s. bspw. Ausubel et al. (1988) "Current Protocols in Molecular Biology", Wiley, New York). Bei diesem Verfahren werden Gesamt-Zellproteine extrahiert, durch Gelelektrophorese aufgetrennt, auf eine Matrix, wie Nitrocellulose, übertragen und mit einer Sonde, wie einem Antikörper, die an das gewünschte Protein spezifisch bindet, inkubiert,. Diese Sonde ist gewöhnlich mit einer chemilumineszierenden oder colorimetrischen Markierung versehen, die sich leicht nachweisen läßt. Das Vorliegen und die beobachtete Menge an Markierung zeigt das Vorliegen und die Menge des gesuchten Mutantenproteins in der Zelle an.

### Beispiel 7: Wachstum von genetisch verändertem Corynebacterium glutamicum - Medien und Anzuchtbedingungen

Genetisch veränderte *Corynebakterien* werden in synthetischen oder natürlichen Wachstumsmedien gezüchtet. Eine Anzahl unterschiedlicher Wachstumsmedien für Corynebakterian sind bekannt und leicht erhältlich (Lieb et al. (1989) Appl. Microbiol. Biotechnol. 32:205-210; von der Osten et al. (1998) Biotechnology Letters 11:11-16; Patent DE 4 120 867; Liebl (1992) "The Genus Corynebacterium", in: The Procaryotes, Bd. II, Balows, A., et al., Hrsg. Springer-Verlag). Diese Medien bestehen aus einer oder mehreren Kohlenstoffquellen, Stickstoffquellen, anorganischen Salzen, Vitaminen und Spurenelementen. Bevorzugte Kohlenstoffquellen sind Zucker, wie Mono-, Di- oder Polysaccharide. Sehr gute Kohlenstoffquellen sind bspw. Glucose, Fructose, Mannose, Galactose, Ribose, Sorbose, Ribulose, Lactose, Maltose, Saccharose, Raffinose, Stärke oder Cellulose. Man kann Zucker auch über komplexe Verbindungen, wie Melassen, oder andere Nebenprodukte der Zucker-Raffinierung zu den Medien geben. Es kann auch vorteilhaft sein, Gemische verschiedener Kohlenstoffquellen zuzugeben. Andere mögliche Kohlenstoffquellen sind Alkohole und organische Säuren, wie Methanol, Ethanol, Essigsäure oder Milchsäure. Stickstoffquellen sind gewöhnlich organische oder anorganische Stickstoffverbindungen oder Materialien, die diese Verbindungen enthalten. Beispielhafte Stickstoffquellen umfassen Ammoniak-Gas oder Ammoniumsalze, wie NH₄Cl oder (NH₄)₂SO₄, NH₄OH, Nitrate, Harnstoff, Aminosäuren oder komplexe Stickstoffquellen, wie Maisquellwasser, Sojamehl, Sojaprotein, Hefeextrakt, Fleischextrakt und andere.

Anorganische Salzverbindungen, die in den Medien enthalten sein können, umfassen die Chlorid-, Phosphor- oder Sulfatsalze von Calcium, Magnesium, Natrium, Kobalt, Molybdän, Kalium, Mangan, Zink, Kupfer und Eisen. Chelatbildner können zum Medium gegeben werden, um die Metallionen in Lösung zu halten. Besonders geeignete Chelatbildner umfassen Dihydroxyphenole, wie Catechol oder Protocatechuat, oder organische Säuren, wie Citronensäure. Die Medien enthalten üblicherweise auch andere Wachstumsfaktoren, wie Vitamine oder Wachstumsförderer, zu denen bspw. Biotin, Riboflavin, Thiamin, Folsäure, Nikotinsäure, Panthothenat und Pyridoxin gehören. Wachstumsfaktoren und Salze stammen häufig von komplexen Medienkomponenten, wie Hefeextrakt, Melassen, Maisquellwasser und dergleichen. Die genaue Zusammensetzung der Medienverbindungen hängt stark vom jeweiligen Experiment ab und wird für jeden spezifischen Fall individuell entschieden. Information über die Medienoptimierung ist erhältlich aus dem Lehrbuch "Applied Microbiol. Physiology, A Practical Approach" (Hrsg. P.M. Rhodes, P.F. Stanbury, IRL Press (1997) S. 53-73, ISBN 0 19 963577 3). Wachstumsmedien lassen sich auch von kommerziellen Anbietern beziehen, wie Standard 1 (Merck) oder BHI (Brain heart infusion, DIFCO) und dergleichen.

Sämtliche Medienkomponenten werden, entweder durch Hitze (20 min bei 1,5 bar und 121°C) oder durch Sterilfiltration, sterilisiert. Die Komponenten können entweder zusammen oder nötigenfalls getrennt sterilisiert werden. Sämtliche Medienkomponenten können zu Beginn der Anzucht zugegen sein oder wahlfrei kontinuierlich oder chargenweise hinzugegeben werden.

Die Anzuchtbedingungen werden für jedes Experiment gesondert definiert. Die Temperatur sollte zwischen 15°C und 45°C liegen und kann während des Experimentes konstant gehalten oder verändert werden. Der pH-Wert des Mediums sollte im Bereich von 5 bis 8,5, vorzugsweise um 7,0 liegen, und kann durch Zugabe von Puffern zu den Medien aufrechterhalten werden. Ein beispielhafter Puffer für diesen Zweck ist ein Kaliumphosphatpuffer. Synthetische Puffer, wie MOPS, HEPES; ACES usw., können alternativ oder gleichzeitig verwendet werden. Der Anzucht-pH-Wert läßt sich während der Anzucht auch durch Zugabe von NaOH oder NH₄OH konstant halten. Werden komplexe Medienkomponenten, wie Hefe-Extrakt, verwendet, sinkt der Bedarf an zusätzlichen Puffern, da viele komplexe Verbindungen eine hohe Pufferkapazität aufweisen. Beim Einsatz eines Fermenters für die Anzucht von Mikroorganismen kann der pH-Wert auch mit gasförmigem Ammoniak reguliert werden.

Die Inkubationsdauer liegt gewöhnlich in einem Bereich von mehreren Stunden bis zu mehreren Tagen. Diese Zeit wird so ausgewählt, daß sich die maximale Menge Produkt in der Brühe ansammelt. Die offenbarten Wachstumsexperimente können in einer Vielzahl von Behältern, wie Mikrotiterplatten, Glasröhrchen, Glaskolben oder Glas- oder Metallfermentern unterschiedlicher Größen durchgeführt werden. Zum Screening einer großen Anzahl von Klonen sollten die Mikroorganismen in Mikrotiterplatten, Glasröhrchen oder Schüttelkolben entweder mit oder ohne Schikanen, gezüchtet werden. Vorzugsweise werden 100-ml-Schüttelkolben verwendet, die mit 10% (bezogen auf das Volumen) des erforderlichen Wachstumsmediums gefüllt sind. Die Kolben sollten auf einem Kreiselschüttler (Amplitude 25 mm) mit einer Geschwindigkeit im Bereich von 100-300 U/min geschüttelt werden. Verdampfungsverluste können durch Aufrechterhalten einer feuchten Atmosphäre verringert werden; alternativ sollte für die Verdampfungsverluste eine mathematische Korrektur durchgeführt werden.

Werden genetisch modifizierte Klone untersucht, sollte auch ein unmodifizierter Kontrollklon oder ein Kontrollklon getestet werden, der das Basisplasmid ohne Insertion enthält. Das Medium wird auf eine OD₆₀₀ von 0,5 - 1,5 angeimpft, wobei Zellen verwendet werden, die auf Agarplatten, wie CM-Platten (10 g/l Glucose, 2,5 g/l NaCl, 2 g/l Harnstoff, 10 g/l Polypepton, 5 g/l Hefeextrakt, 5 g/l Fleischextrakt, 22 g/l Agar pH-Wert 6,8 mit 2 M NaOH), die bei 30°C inkubiert worden sind, gezüchtet wurden. Das Animpfen der Medien erfolgt entweder durch Einbringen einer Kochsalzlösung von C. *glutamicum*-Zellen von CM-Platten oder durch Zugabe einer flüssigen Vorkultur dieses Bakteriums.

### Beispiel 8: In-vitro-Analyse der Funktion mutanter Proteine

Die Bestimmung der Aktivitäten und kinetischen Parameter von Enzymen ist im Fachgebiet gut bekannt. Experimente zur Bestimmung der Aktivität eines bestimmten veränderten Enzyms müssen an die spezifische Aktivität des Wildtypenzyms angepaßt werden, was innerhalb der Fähigkeiten des Fachmann liegt. Überblicke über Enzyme im allgemeinen sowie spezifische Einzelheiten, die die Struktur, Kinetiken, Prinzipien, Verfahren, Anwendungen und Beispiele zur Bestimmung vieler Enzymaktivitäten betreffen, können bspw. in den nachstehenden Literaturstellen gefunden werden: Dixon, M., und Webb, E.C: (1979) Enzymes, Longmans, London; Fersht (1985) Enzyme Structure and Mechanism, Freeman, New York; Walsh (1979) Enzymatic Reaction Mechanisms. Freeman, San Francisco; Price, N.C., Stevens, L. (1982) Fundamentals of Enzymology. Oxford Univ. Press: Oxford; Boyer, P.D: Hrsg. (1983) The Enzymes, 3. Aufl., Academic Press, New York; Bisswanger, H. (1994) Enzymkinetik, 2. Aufl. VCH, Weinheim (ISBN 3527300325); Bergmeyer, H.U., Bergmeyer, J., Graßl, M. Hrsg. (1983-1986) Methods of Enzymatic Analysis, 3. Aufl. Bd. I-XII, Verlag Chemie: Weinheim; und Ullmann's Encyclopedia of Industrial Chemistry (1987) Bd. A9, "Enzymes", VCH, Weinheim, S. 352-363.

Die Aktivität von Proteinen, die an DNA binden, kann durch viele gut eingeführte Verfahren gemessen werden, wie DNA-Banden-Shift-Assays (die auch als Gelretardations-Assays bezeichnet werden). Die Wirkung dieser Proteine auf die Expression anderer Moleküle kann mit Reportergen-Assays (wie in Kolmar, H. et al., (1995) EMBO J. 14:3895-3904 und den darin zitierten Literaturstellen beschrieben) gemessen werden. Reportergen-Testsysteme sind wohlbekannt und für Anwendungen in pro- und eukaryotischen Zellen etabliert, wobei Enzyme, wie beta-Galactosidase, Grün-Fluoreszenz-Protein und mehrere andere verwendet werden.

Die Bestimmung der Aktivität von Membran-Transportproteinen kann gemäß Techniken, wie sie in Gennis, R.B. (1989) "Pores, Channels and Transporters", in Biomembranes, Molecular Structure and Function, Springer: Heidelberg, S. 85-137; 199-234; und 270-322 beschrieben sind, erfolgen.

### Beispiel 9: Analyse des Einflusses von mutiertem Protein auf die Produktion des gewünschten Produktes

Die Wirkung der genetischen Modifikation in *C*. *glutamicum* auf die Produktion einer gewünschten Verbindung (wie einer Aminosäure) kann bestimmt werden, indem die modifizierten Mikroorganismen unter geeigneten Bedingungen (wie den vorstehend beschriebenen) gezüchtet werden und das Medium und/oder die zellulären Komponenten bezüglich der erhöhten Produktion des gewünschten Produktes (d.h. einer Aminosäure) untersucht wird/werden. Solche Analysetechniken sind dem Fachmann wohlbekannt und umfassen Spektroskopie, Dünnschichtchromatographie, Färbeverfahren verschiedener Art, enzymatische und mikrobiologische Verfahren sowie analytische Chromatographie, wie Hochleistungs-Flüssigkeitschromatographie (s. bspw. Ullman, Encyclopedia of Industrial Chemistry, Bd. A2, S. 89-90 und S. 443-613, VCH: Weinheim (1985); Fallon, A., et al., (1987) "Applications of HPLC in Biochemistry" in: Laboratory Techniques in Biochemistry and Molecular Biology, Bd. 17; Rehm et al. (1993) Biotechnology, Bd. 3, Kapitel III: "Product recovery and purification", S. 469-714, VCH: Weinheim; Belter, P.A. et al. (1988) Bioseparations: downstream processing for Biotechnology, John Wiley and Sons; Kennedy, J.F. und Cabral, J.M.S. (1992) Recovery processes for biological Materials, John Wiley and Sons; Shaeiwitz, J.A. und Henry, J.D. (1988) Biochemical Separations, in Ullmann's Encyclopedia of Industrial Chemistry, Bd. B3; Kapitel 11, S. 1-27, VCH: Weinheim; und Dechow, F.J. (1989) Separation and purification techniques in biotechnology, Noyes Publications).

Zusätzlich zur Messung des Fermentationsendproduktes ist es ebenfalls möglich, andere Komponenten der Stoffwechselwege zu analysieren, die zur Produktion der gewünschten Verbindung verwendet werden, wie Zwischen- und Nebenprodukte, um die Gesamt-Effizienz der Produktion der Verbindung zu bestimmen. Die Analyseverfahren umfassen Messungen der Nährstoffmengen im Medium (bspw. Zucker, Kohlenwasserstoffe, Stickstoffquellen, Phosphat und andere Ionen), Messungen der Biomassezusammensetzung und des Wachstums, Analyse der Produktion gemeinsamer Metabolite von Biosynthesewegen und Messungen von Gasen, die während der Fermentation erzeugt werden. Standardverfahren für diese Messungen sind in Applied Microbial Physiology; A Practical Approach, P.M. Rhodes und P.F. Stanbury, Hrsg. IRL Press, S. 103-129; 131-163 und 165-192 (ISBN: 0199635773) und den darin angegebenen Literaturstellen beschrieben.

### Beispiel 10: Reinigung des gewünschten Produktes aus C. glutamicum-Kultur

Die Gewinnung des gewünschten Produktes aus *C. glutamicum*-Zellen oder aus dem Überstand der vorstehend beschriebenen Kultur kann durch verschiedene, im Fachgebiet bekannte Verfahren erfolgen. Wird das gewünschte Produkt von den Zellen nicht sezerniert, können die Zellen aus der Kultur durch langsame Zentrifugation geerntet werden, die Zellen können durch Standard-Techniken, wie mechanische Kraft oder Ultraschallbehandlung, lysiert werden. Die Zelltrümmer werden durch Zentrifugation entfernt, und die Überstandsfraktion, die die löslichen Proteine enthält, wird zur weiteren Reinigung der gewünschten Verbindung erhalten. Wird das Produkt von den *C. glutamicum*-Zellen sezerniert, werden die Zellen durch langsame Zentrifugation aus der Kultur entfernt, und die Überstandsfraktion wird zur weiteren Reinigung behalten.

Die Überstandsfraktion aus beiden Reinigungsverfahren wird einer Chromatographie mit einem geeigneten Harz unterworfen, wobei das gewünschte Molekül entweder auf dem Chromatographieharz zurückgehalten wird, viele Verunreinigungen in der Probe jedoch nicht, oder die Verunreinigungen auf dem Harz zurückbleiben, die Probe hingegen nicht. Diese Chromatographieschritte können nötigenfalls wiederholt werden, wobei die gleichen oder andere Chromatographieharze verwendet werden. Der Fachmann ist in der Auswahl der geeigneten Chromatographieharze und ihrer wirksamsten Anwendung für ein bestimmtes zu reinigendes Molekül bewandert. Das gereinigte Produkt kann durch Filtration oder Ultrafiltration konzentriert und bei einer Temperatur aufbewahrt werden, bei der die Stabilität des Produktes maximal ist.

Im Fachgebiet sind viele Reinigungsverfahren bekannt, und das vorhergehende Reinigungsverfahren soll nicht einschränkend sein. Diese Reinigungstechniken sind bspw. beschrieben in Bailey, J.E. & Ollis, D.F. Biochemical Engineering Fundamentals, McGraw-Hill: New York (1986).

Die Identität und Reinheit der isolierten Verbindungen kann durch Techniken des Standes der Technik bestimmt werden. Diese umfassen Hochleistungs-Flüssigkeitschromatographie (HPLC), spektroskopische Verfahren, Färbeverfahren, Dünnschichtchromatographie, NIRS, Enzymtest oder mikrobiologische Tests. Diese Analyseverfahren sind zusammengefaßt in: Patek et al. (1994) Appl. Environ. Microbiol. 60:133-140; Malakhova et al. (1996) Biotekhnologiya 11 27-32; und Schmidt et al. (1998) Bioprocess Engineer. 19:67-70. Ulmann's Encyclopedia of Industrial Chemistry (1996) Bd. A27, VCH: Weinheim, S. 89-90, S. 521-540, S. 540-547, S. 559-566, 575-581 und S. 581-587; Michal, G (1999) Biochemical Pathways: An Atlas of Biochemistry and Molecular Biology, John Wiley and Sons; Fallon, A. et al. (1987) Applications of HPLC in Biochemistry in: Laboratory Techniques in Biochemistry and Molecular Biology, Bd. 17.

### Äquivalente

Der Fachmann erkennt oder kann - indem er lediglich Routineverfahren verwendet - viele Äquivalente der erfindungsgemäßen spezifischen Ausführungsformen feststellen.

Die Angaben in Tabelle 1 sind folgendermassen zu verstehen:

In Spalte 1"DNA-ID" bezieht sich die jeweilige Zahl auf die SEQ ID NO des anhängenden Sequenzprotokolls. Eine "1" in der Spalte "DNA-ID" bedeutet demzufolge ein Verweis auf SEQ ID NO:1.

In Spalte 2"AS-ID" bezieht sich die jeweilige Zahl auf die SEQ ID NO des anhängenden Sequenzprotokolls. Eine "2" in der Spalte "AS-ID" bedeutet demzufolge ein Verweis auf SEQ ID NO:2.

In Spalte 3"Identifikation" wird eine eindeutige interne Bezeichnung für jede Sequenz aufgeführt.

In Spalte 4 "AS-POS" bezieht sich die jeweilige Zahl auf die Aminosäureposition der Polypeptidsequenz "AS-ID" in der gleichen Zeile. Eine "26" in der Spalte "AS-POS" bedeutet demzufolge die Aminosäureposition 26 der entsprechend angegebenen Polypeptidsequenz. Die Zählung der Position beginnt N-Terminal bei +1.

In Spalte 5 "AS-Wildtyp" bezeichnet der jeweilige Buchstabe die Aminosäure - dargestellt im Ein-Buchstaben-Code- an der in Spalte 4 angegebenen Position beim entsprechenden Wildtyp-Stamm.

In Spalte 6 "AS-Mutante" bezeichnet der jeweilige Buchstabe die Aminosäure - dargestellt im Ein-Buchstaben-Code- an der in Spalte 4 angegebenen Position beim entsprechenden Mutanten-Stamm.

In Spalte 7 "Funktion" wird die physiologische Funktion der entsprechenden Polypeptidsequenz aufgeführt.

### Ein-Buchstaben-Code der proteinogenen Aminosäuren:

- A: Alanin
- C: Cystein
- D: Aspartat
- E: Glutamat
- F: Phenylalanin
- G: Glycin
- H: His
- I: Isoleucin
- K: Lysin
- L: Leucin
- M: Methionin
- N: Asparagin
- P: Prolin
- Q: Glutamin
- R: Arginin
- S: Serin
- T: Threonin
- V: Valin
- W: Tryptophan
- Y: Tyrosin

**Tabelle 1 Gene die für Kohlenstoffmetabolismus- und Energieproduktion-Proteine codieren**

| **DNA ID:** | **AS ID:** | **Identifikation:** | **AS Pos:** | **AS Wildtyp** | **AS Mutante** | **Funktion:** |
|---|---|---|---|---|---|---|
| 1 | 2 | RXA00149 | 75 | P | S | METHYLMALONYL-COA MUTASE (EC 5.4.89.2) |
| | | | 413 | G | E | METHYLMALONYL-COA MUTASE (EC 5.4.99.2) |
| | | | 417 | A | V | METHYLMALONYL-COA MUTASE (EC 5.4.99.2) |

## Patentansprüche

1. Ein isoliertes Nukleinsäuremolekül, kodierend für ein Polypeptid mit der Aminosäure SEQ ID NO:2, wobei im Polypeptid die Aminosäureposition 75 durch Serin ersetzt ist, die Aminosäureposition 413 durch Glutamat und/oder die Aminosäureposition 417 durch Valin ersetzt ist.

2. wie ursprünglicher Anspruch 3 [Ein Vektor der wenigstens eine Nukleinsäuresequenz nach Anspruch 1 enthält.]

3. Eine Wirtszelle, die einen Vektor nach Anspruch 2 enthält

4. Eine Wirtszelle nach Anspruch 3, wobei die Expression des besagten Nukleinsäuremoleküls zur Modulation der Produktion einer Aminosäure, insbesondere Lysin, aus besagter Zelle führt.

5. Verfahren zur Herstellung einer Aminosäure, insbesondere Lysin, welches die Kultivierung einer Zelle beinhaltet, die mit wenigstens einem Vektor nach Anspruch 2 transfiziert worden ist, so dass die Aminosäure, insbesondere Lysin, produziert wird.

## Claims

1. An isolated nucleic acid molecule, coding for a polypeptide with amino acid SEQ ID NO. 2 wherein the amino acid position 75 of the polypeptide is replaced by serine, wherein the amino acid position 413 is replaced by glutamate and/or wherein the amino acid position 417 is replaced by valine.

2. A vector, which comprises at least one nucleic acid sequence as claimed in claim 1.

3. A host cell, which comprises a vector according to claim 2.

4. A host cell of claim 3, wherein the expression of said nucleic acid molecule leads to modulation of production of an amino acid, in particular lysine in said cell.

5. A method for preparing an amino acid, particularly lysine, which comprises culturing a cell which has been transfected with at least one vector as claimed in claim 2, so that the amino acid, particularly lysine, is produced.

## Revendications

1. Molécule isolée d'acide nucléique codant un polypeptide avec la séquence d'acide aminés SEQ ID: N02, la position d'acide aminé 75 dans le polypeptide étant remplacée par la sérine, la position d'acide aminé 413 étant remplacée par le glutamate et/ou la position d'acide aminé 417 étant remplacée par la valine.

2. Vecteur qui contient au moins une séquence d'acide nucléique selon la revendication 1.

3. Cellule hôte qui contient un vecteur selon la revendication 2.

4. Cellule hôte selon la revendication 3, l'expression de ladite molécule d'acide nucléique permettant de moduler la production d'un acide aminé, plus particulièrement de la lysine, à partir de ladite cellule.

5. Procédé de fabrication d'un acide aminé, plus particulièrement de la lysine, qui comprend la culture d'une cellule transfectée avec au moins un vecteur selon la revendication 2 de façon à produire l'acide aminé, plus particulièrement la lysine.
